(19) Europäisches Patentamt

European Patent Office

Office européen des brevets

(11) **EP 2 438 197 B1**

(12) **EUROPEAN PATENT SPECIFICATION**

(45) Date of publication and mention of the grant of the patent:
**15.07.2015 Bulletin 2015/29**

(51) Int Cl.:
**C12Q 1/68** (2006.01)

(21) Application number: **10784236.1**

(22) Date of filing: **07.06.2010**

(86) International application number:
**PCT/US2010/037659**

(87) International publication number:
**WO 2010/141955 (09.12.2010 Gazette 2010/49)**

(54) **METHODS OF DETECTING CANCER**

**VERFAHREN ZUR KREBSERKENNUNG**

**PROCÉDÉ DE DÉTECTION DU CANCER**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **05.06.2009 US 184685 P**

(43) Date of publication of application:
**11.04.2012 Bulletin 2012/15**

(73) Proprietor: **Myriad Genetics, Inc.**
**Salt Lake City, UT 84108 (US)**

(72) Inventors:
• **GUTIN, Alexander**
  **Salt Lake City**
  **Utah 84108 (US)**
• **LANCHBURY, Jerry**
  **Salt Lake City**
  **Utah 84108 (US)**
• **WAGNER, Susanne**
  **Salt Lake City**
  **Utah 84108 (US)**
• **ABKEVICH, Victor**
  **Salt Lake City**
  **Utah 84108 (US)**

(74) Representative: **Vossius & Partner**
**Patentanwälte Rechtsanwälte mbB**
**Siebertstrasse 3**
**81675 München (DE)**

(56) References cited:
**WO-A2-2009/049166**

• **DING ET AL.: "Somatic mutations affect key pathways in lung adenocarcinoma.", NATURE, vol. 455, no. 7216, 23 October 2008 (2008-10-23), pages 1069-1075, XP002597660,**
• **FRANCESCA ANDRIANI ET AL: "Detecting lung cancer in plasma with the use of multiple genetic markers", INTERNATIONAL JOURNAL OF CANCER, vol. 108, no. 1, 1 January 2004 (2004-01-01), pages 91-96, XP55040284, ISSN: 0020-7136, DOI: 10.1002/ijc.11510**
• **LILLEBERG S L ET AL: "High sensitivity scanning of colorectal tumors and matched plasma DNA for mutations in APC, TP53, K-RAS, and BRAF genes with a novel DHPLC fluorescence detection platform", NEW YORK ACADEMY OF SCIENCES. ANNALS, WILEY-BLACKWELL PUBLISHING, INC, US, vol. 1022, 1 June 2004 (2004-06-01), pages 250-256, XP002392437, ISSN: 0077-8923, DOI: 10.1196/ANNALS.1318.039 ISBN: 978-1-57331-623-1**
• **DIEHL F ET AL: "Analysis of Mutations in DNA Isolated From Plasma and Stool of Colorectal Cancer Patients", GASTROENTEROLOGY, ELSEVIER, PHILADELPHIA, PA, vol. 135, no. 2, 1 August 2008 (2008-08-01), pages 489-498.e7, XP023901355, ISSN: 0016-5085, DOI: 10.1053/J.GASTRO.2008.05.039 [retrieved on 2008-08-01]**
• **SJÖBLOM TOBIAS ET AL: "The consensus coding sequences of human breast and colorectal cancers", SCIENCE, AMERICAN ASSOCIATION FOR THE ADVANCEMENT OF SCIENCE, WASHINGTON, DC; US, vol. 314, no. 5797, 13 October 2006 (2006-10-13), pages 268-274, XP002478760, ISSN: 0036-8075, DOI: 10.1126/SCIENCE.1133427**

**(Cont. next page)**

- MARK R. TRUSHEIM ET AL: "Stratified medicine: strategic and economic implications of combining drugs and clinical biomarkers", NATURE REVIEWS DRUG DISCOVERY, vol. 6, no. 4, 1 April 2007 (2007-04-01), pages 287-293, XP55040333, ISSN: 1474-1776, DOI: 10.1038/nrd2251
- STINE A. DANIELSEN ET AL: "Novel mutations of the suppressor gene PTEN in colorectal carcinomas stratified by microsatellite instability- and TP53 mutation- status", HUMAN MUTATION, vol. 29, no. 11, 1 November 2008 (2008-11-01), pages E252-E262, XP55040456, ISSN: 1059-7794, DOI: 10.1002/humu.20860
- M. MORONI ET AL: "Somatic mutation of EGFR catalytic domain and treatment with gefitinib in colorectal cancer", ANNALS OF ONCOLOGY, vol. 16, no. 11, 1 November 2005 (2005-11-01), pages 1848-1849, XP55040454, ISSN: 0923-7534, DOI: 10.1093/annonc/mdi356
- DING ET AL.: 'Somatic mutations affect key pathways in lung adenocarcinoma.' NATURE vol. 455, no. 7216, 23 October 2008, pages 1069 - 1075, XP002597660
- WOOD LAURA D ET AL: "The genomic landscapes of human breast and colorectal cancers.", SCIENCE (NEW YORK, N.Y.) 16 NOV 2007, vol. 318, no. 5853, 16 November 2007 (2007-11-16), pages 1108-1113, ISSN: 1095-9203

**Description**

**CROSS REFERENCE TO RELATED APPLICATIONS**

This application claims the priority benefit of U.S. Provisional Application Serial No. 61/184,685 (filed on June 5, 2009).

**FIELD OF THE INVENTION**

The invention generally relates to a molecular classification of disease and particularly to molecular markers for cancer and methods of use thereof.

**BACKGROUND OF THE INVENTION**

Cancer is a major health challenge. Nearly 560,000 people die from cancer annually in the United States alone, representing almost 23% of all deaths. Despite recent advances in molecular and imaging diagnostics, one of the most vexing aspects of cancer remains early detection. In fact, for certain types of cancer-*e.g.*, pancreatic adenocarcinoma-detection often occurs so late as to practically preclude any good prognosis. Thus there is an urgent need for sensitive methods of detecting cancer.

**SUMMARY OF THE INVENTION**

Mutations in certain genes are associated with cancer in general and with specific cancer types. For example, inactivating mutations in the *TP53* gene are found in approximately 50% of all solid tumors and activating mutations in the *KRAS* or *BRAF* genes are often found in colorectal cancer. It has been discovered that screening patients for mutations in certain genes can detect and classify cancer. More specifically, it has been determined that (a) screening certain genes (*e.g., APC, EGFR, KRAS, PTEN,* and *TP53)* for mutations will detect nearly 95% of all cancers, while (b) screening certain genes *(e.g., AIM1, APC, CDKN2A, EGFR, FBN2, FBXW7, FLJ13479, IDH1, KRAS, PIK3CA, PIK3R1, PTEN, RB1, SMAD4, TGFBR2, TNN,* and *TP53)* for mutations can accurately classify the cancer (e.g., as breast cancer, colon cancer, glioblastoma, pancreatic cancer, etc.).

Thus the invention generally provides methods comprising analyzing panels of genes from a sample obtained from a patient (*e.g.*, mRNA or cDNA synthesized therefrom) and determining the mutational status of the genes in the panel, wherein the presence of a particular mutational status in particular genes in the panel indicates (a) the patient has cancer and/or (b) the patient has a particular cancer.

One aspect of the invention provides a method of detecting mutations comprising: (1) analyzing in a bodily fluid sample from a human subject a panel of genes consisting of between 5 and 5,000 genes, wherein said panel comprises at least five genes chosen from the group consisting of the genes listed in Table 1; and (2) determining whether any of the genes in Table 1 harbors a mutation.

The panel comprises the *APC, EGFR, KRAS, PTEN,* and *TP53* genes. In some embodiments the panel comprises the genes listed in Table 3. In some embodiments the panel comprises the genes listed in Table 2. In some embodiments the panel comprises the genes listed in Table 1.

One aspect of the invention provides a method of detecting cancer comprising: (1) analyzing a panel of genes comprising the *APC, EGFR, KRAS, PTEN,* and *TP53* genes in a bodily fluid sample; and (2) determining whether any of the *APC, EGFR, KRAS, PTEN,* or *TP53* genes harbors a mutation; wherein said mutation indicates the presence of cancer.

In some embodiments the panel comprises the genes listed in Table 3. In some embodiments the panel comprises the genes listed in Table 2. In some embodiments the panel comprises the genes listed in Table 1. In some embodiments the mutation is selected from the group consisting of those listed in Table 7 and/or Table 8.

One aspect of the invention provides a method of determining the likelihood a patient has cancer $c_1$ comprising: (1) analyzing in a fluid sample a panel of genes comprising the genes listed in Table 3; (2) detecting a mutation in at least one of said genes listed in Table 3; and (3) calculating a likelihood said patient has cancer $c_1$ using the formula: $P(c_1|g_1, g_2, ..., g_n) = P_0(c_1) \Pi_i M(g_i|\ c_1) / \sum_t P_0(t) \Pi_i M(g_i|t)$; wherein the product is taken over all genes in said panel mutated in the sample ($i=1, 2, ..., n$), the sum is taken over all cancer types $t$, $M(g|\ c_1)$ is the frequency of somatic mutations in gene g in cancer type $c_1$, and $P_0(c_1)$ is the *a priori* probability of cancer $c_1$ given that the patient has a cancer.

In some embodiments such method further comprises calculating a likelihood said patient has a second cancer $c_2$ using the formula: $P(c_2|g_1, g_2, ..., g_n) = P_0(c_2) \Pi_i M(g_i|\ c_2) / \sum_t P_0(t) \Pi_i M(g_i|t)$; wherein the product is taken over all genes in said panel mutated in the sample ($i=1, 2, ..., n$), the sum is taken over all cancer types $t$, $M(g|\ c_2)$ is the frequency of somatic mutations in gene g in cancer type $c_2$, and $P_0(c_2)$ is the *a priori* probability of cancer type $c_2$ given that the

patient has a cancer.

Some embodiments further comprise recommending, prescribing, ordering, or performing a test for the presence of cancer $c_1$ in said patient. In some embodiments the test for the presence of cancer $c_1$ is recommended, prescribed, ordered, or performed if the calculated likelihood said patient has said cancer $c_1$ is above a threshold value (e.g., 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99%, or 100%).

In some embodiments the test for the presence of cancer $c_1$ is recommended, prescribed, ordered, or performed if the calculated likelihood said patient has said cancer $c_1$ is higher than the calculated likelihood said patient has cancer $c_2$. In some embodiments the method further comprises recommending, prescribing, ordering, or performing a test for the presence of cancer $c_2$ in said patient if said test for the presence of cancer $c_1$ does not indicate the presence of cancer $c_1$.

In various embodiments of the invention the said bodily fluid sample is a blood sample. In some embodiments the blood sample is a plasma sample. In some embodiments the blood sample is a serum sample.

In some embodiments detecting a mutation or determining whether a gene harbors a mutation comprises analyzing an mRNA molecule from a sample or analyzing a DNA molecule synthesized using the mRNA molecule as a template. In some embodiments detecting a mutation or determining whether a gene harbors a mutation comprises analyzing a nucleic acid from a sample by a technique chosen from resequencing, TaqMan™, microarray analysis, and FISH.

In some embodiments nucleic acids to be analyzed are derived from an extracellular vesicle. In some embodiments such extracellular vesicle is an exosome.

One aspect of the invention provides a kit comprising reagents for analyzing a panel of genes consisting of between 5 and 5,000 genes, said kit comprising reagents for detecting mutations in at least five genes selected from the group consisting of the genes listed in Table 1. The kit comprises reagents for detecting mutations in the *APC, EGFR, KRAS, PTEN,* and *TP53* genes.E3. In some embodiments the kit comprises reagents for detecting mutations in the genes listed in Table 3. In some embodiments the kit comprises reagents for detecting mutations in the genes listed in Table 2. In some embodiments the kit comprises reagents for detecting mutations in the genes listed in Table 1.

Unless otherwise defined, all technical and scientific terms used herein have the same meaning as commonly understood by one of ordinary skill in the art to which this invention pertains. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

Other features and advantages of the invention will be apparent from the following detailed description, and from the claims.

## BRIEF DESCRIPTION OF THE DRAWINGS

FIG.1 illustrates the sensitivity of a panel of five genes for detecting cancer.

FIG.2 illustrates one embodiment of the invention using various biomarkers to determine which specific cancer is present in a patient.

FIG.3 illustrates example mutation frequencies in various cancers.

FIG.4 illustrates example cancer rates based on cancer site and gender.

FIG.5 shows the detection of mutations in exosomes from cancer serum samples.

## DETAILED DESCRIPTION OF THE INVENTION

Mutations in certain genes are associated with cancer in general and with specific cancer types. For example, inactivating mutations in the *TP53* gene are found in approximately 50% of all solid tumors and activating mutations in the *KRAS* or *BRAF* genes are often found in colorectal cancer.

The invention is based in part on the discovery that analyzing patient samples for mutations in a relatively small number of genes can (a) detect the vast majority of cancers and (b) specify in which tissue the cancer is located. More specifically, it has been determined that (a) screening certain genes *(e.g.,* the genes listed in Table 4 below) for mutations will detect cancer *(e.g.,* nearly 95% of all cancers), while (b) screening certain genes *(e.g.,* the genes listed in Tables 2 & 3 below) for mutations can accurately classify the cancer (e.g., as breast cancer, colon cancer, glioblastoma, pancreatic cancer, etc.).

Thus the invention provides a method of detecting mutations comprising (1) analyzing a panel of genes consisting of between 5 and 5,000 genes in a bodily fluid sample from a human subject, wherein said panel comprises at least five genes chosen from the group consisting of the genes listed in Table 1; and (2) determining whether at least one of said five genes harbors a mutation.

In some embodiments the panel consists of between 5 and 4,500, between 5 and 4,000, between 5 and 3,500, between 5 and 3,000, between 5 and 2,500, between 5 and 2,000, between 5 and 1,500, between 5 and 1,000, between 5 and 500, between 5 and 400, between 5 and 300, between 5 and 200, between 5 and 150, between 5 and 100, between 5 and 75, or between 5 and 50 genes. In some embodiments the genes chosen from Table 1 comprise at least 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99% or 100% of the panel.

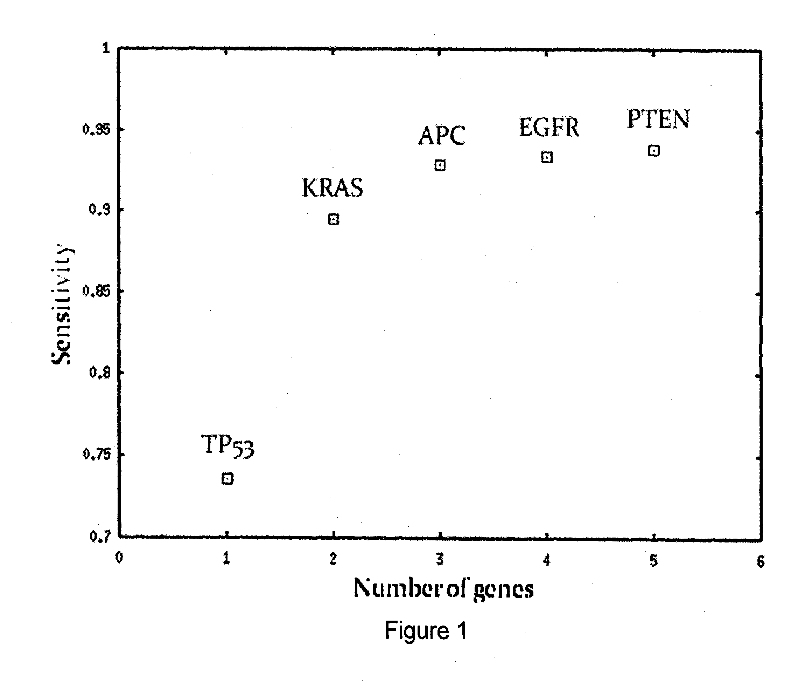

It has been discovered that screening patient samples for mutations in the genes listed in Table 1 below will detect the vast majority of cancers. In Example 2, for instance, screening for mutations in the *APC, EGFR, KRAS, PTEN* and *TP53* genes is shown to detect nearly 95% of cancers (FIG.1). Analyzing the remaining genes in Table 1 will detect many of the remaining cancers. Thus one aspect of the invention provides a method of detecting cancer comprising: (1) analyzing a panel of genes in a bodily fluid sample from a human subject, wherein said panel comprises at least five genes chosen from the group consisting of the genes listed in Table 1; and (2) determining whether at least one of said five genes harbors a mutation; wherein said mutation indicates the presence of cancer. In some embodiments the mutation is chosen from those listed in Table 7 and/or Table 8.

In some embodiments of this aspect the panel consists of between 5 and 4,500, between 5 and 4,000, between 5 and 3,500, between 5 and 3,000, between 5 and 2,500, between 5 and 2,000, between 5 and 1,500, between 5 and 1,000, between 5 and 500, between 5 and 400, between 5 and 300, between 5 and 200, between 5 and 150, between 5 and 100, between 5 and 75, or between 5 and 50 genes. In some embodiments the genes chosen from Table 1 comprise at least 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99% or 100% of the panel.

It has further been discovered that one can detect a particular cancer c in a patient by screening for somatic mutations in *n* genes $g_1, g_2, ..., g_n$ in the sample and applying the following equation:

$$P(c|g_1, g_2, ..., g_n) = P_0(c) \; \Pi_i \, M(g_i|c) \; / \; \Sigma_t \, P_0(t) \; \Pi_i \, M(g_i|t) \qquad (1)$$

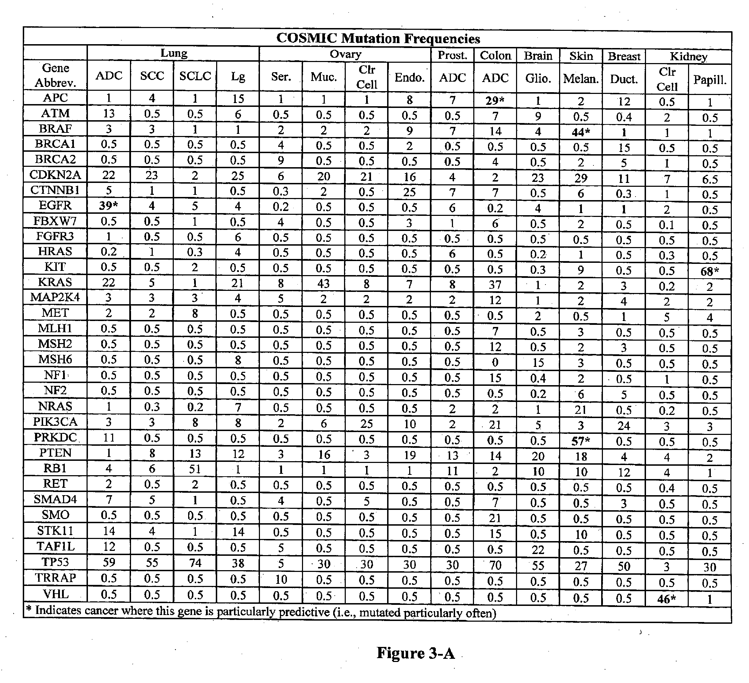

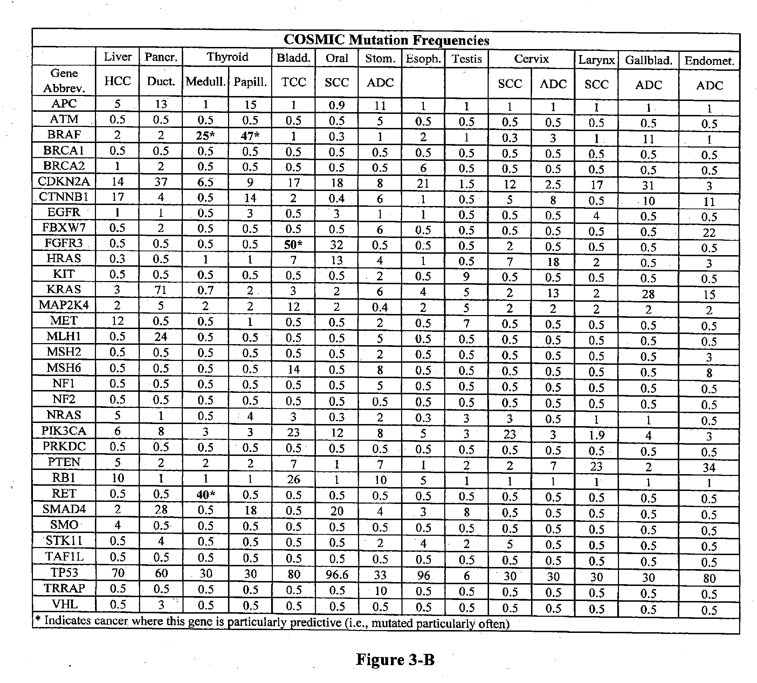

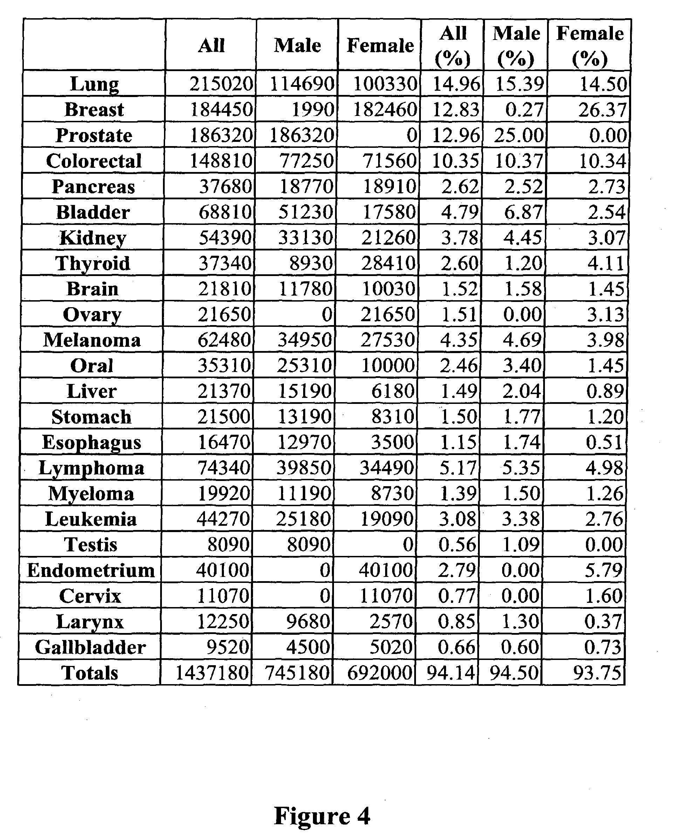

where the product is taken over all genes mutated in the sample (*i=1, 2, ..., n*) and the sum is taken over all cancer types *t*. See Example 1, *infra. M(g|c)* is the frequency of somatic mutations in gene g in cancer type *c. See, e.g.,* FIG.3. $P_0(c)$ is the *a priori* probability of cancer type c given that the patient has a cancer. *See* FIG.4.

Note that the reference values discussed herein (e.g., frequency of mutations in any particular gene in any particular cancer type and probability of a particular cancer type given the patient has cancer) may be tailored to suit the needs of the skilled artisan. For example, mutation frequencies and the relative prevalence of particular cancer types may vary between, e.g., ethnic populations, countries, regions, etc. FIGs 3 & 4 therefore present non-limiting examples of how such values may be obtained and used in the methods of the invention.

Thus one aspect of the invention provides a method of determining the likelihood a patient has a particular cancer $c_1$ comprising:

(1) analyzing a panel of genes in a bodily fluid sample from a human subject, wherein said panel comprises the genes listed in Table 3;
(2) determining whether the genes listed in Table 3 harbor a mutation;
(3) calculating a likelihood said patient has cancer $c_1$ using the formula: $P(c_1|g_1, g_2, ..., g_n) = P_0(c_1) \, \Pi_i \, m(g_i| \, c_1) \, / \, \Sigma_t \, P_0(t) \, \Pi_i \, M(g_i|\, t)$; wherein the product is taken over all genes in said panel mutated in the sample (*i=1, 2, ..., n*), the sum is taken over all cancer types *t, M(g| $c_1$)* is the frequency of somatic mutations in gene *g* in cancer type $c_1$, and $P_0(c_1)$ is the *a priori* probability of cancer $c_1$ given that the patient has a cancer.

As used herein, the *"a priori* probability of cancer c given that the patient has a cancer" refers to the general incidence of the particular cancer *c* in the relevant cancer patient population (*e.g.,* males or females). In other words, this is the relative proportion of all cancers in the relevant population represented by the particular cancer *c*. Such incidences may be gathered from various sources-*e.g.,* yearly American Cancer Society reports on cancer incidence (as in Example 1, *infra*), which often give detailed breakdowns of specific cancer incidence in relevant patient subpopulations such as male vs. female, race or ethnicity, etc.

In some embodiments it is concluded that the patient has a particular cancer $c_1$ only if the calculated likelihood said patient has said cancer $c_1$ is above a threshold value. This threshold value may be arbitrarily chosen *(e.g.,* 95% probability is good enough) or determined empirically *(e.g.,* patients with a calculated probability above 80% have ended up with the particular cancer with enough frequency to validate this as a good threshold). In some embodiments said threshold value is chosen from the group consisting of 35%, 40%, 45%, 50%, 55%, 60%, 65%, 70%, 75%, 80%, 85%, 90%, 95%, 96%, 97%, 98%, and 99%.

Since some organs can develop cancers of different types (such as adenocarcinoma and squamous cell carcinoma in lung), one may calculate the probability *P(o)* that the cancer has developed in organ *o*:

$$P(o|g_1, g_2, ..., g_n) = \Sigma_c \, P(c|g_1, g_2, ..., g_n) \qquad (2)$$

where the sum is over all cancer types c of the organ *o*. Using Equation (2), the probabilities are calculated for each organ *o*, and the organ with the highest probability is the most likely cancer site in the patient. The patient may then optionally be examined by additional diagnostic techniques to confirm cancer site. If the most likely cancer site is not confirmed, the organ with the second highest probability may then be examined and so on.

Thus one aspect of the invention provides a method of diagnosing cancer in a particular organ $o_1$ comprising:

(1) determining the mutational status of a panel of genes;
(2) calculating a likelihood $P(o_1)$ said patient has a cancer in organ $o_1$ using the formula:

$$P(o|g_1, g_2, ..., g_n) = \Sigma_c \, P(c|g_1, g_2, ..., g_n)$$

wherein the sum is taken over all cancer types *c* of the organ $o_1$, and *P(c)* is calculated using the formula:

$$P(c_1|g_1, g_2, ..., g_n) = P_0(c_1) \, \Pi_i \, M(g_i|\,c_1) \, / \, \Sigma_t \, P_0(t) \, \Pi_i \, M(g_i|t)$$

wherein the product is taken over all genes in said panel mutated in the sample (*i=1, 2, ..., n*), the sum is taken over all cancer types *t*, $M(g|\,c_1)$ is the frequency of somatic mutations in gene *g* in cancer type $c_1$, and $P_0(c_1)$ is the *a priori* probability of cancer $c_1$ given that the patient has a cancer.

When screening a patient for cancer *(e.g.,* early detection), it will often be desirable to calculate the probabilities of several different cancers *(e.g.,* the most prevalent cancers in the relevant patient population or the cancers listed in Tables 3 & 4) so as to allow comparison to determine which of a plurality of cancers is the most likely. Thus another aspect of the invention provides a method of determining the likelihood a patient has a particular cancer $c_1$ comprising:

(1) determining the mutational status of a panel of genes;
(2) calculating a likelihood $P(c_1)$ said patient has a first cancer $c_1$ using the formula:

$$P(c_1|g_1, g_2, ..., g_n) = P_0(c_1) \, \Pi_i \, M(g_i|\,c_1) \, / \, \Sigma_t \, P_0(t) \, \Pi_i \, M(g_i|t)$$

wherein the product is taken over all genes in said panel mutated in the sample *(i=1,2,...,n)*, the sum is taken over all cancer types *t*, $M(g|\,c_1)$ is the frequency of somatic mutations in gene g in cancer type $c_1$, and $P_0(c_1)$ is the *a priori* probability of cancer type $c_1$ given that the patient has a cancer; and (3) calculating a likelihood $P(c_2)$ said patient has a second cancer $c_2$ using the formula:

$$P(c_2|g_1, g_2, ..., g_n) = P_0(c_2) \, \Pi_i \, M(g_i|\,c_2) \, / \, \Sigma_t \, P_0(t) \, \Pi_i \, M(g_i|t)$$

wherein the product is taken over all genes in said panel mutated in the sample (*i=1, 2, ..., n*), the sum is taken over all cancer types *t*, $M(g|\,c_2)$ is the frequency of somatic mutations in gene *g* in cancer type $c_2$, and $P_0(c_2)$ is the *a priori* probability of cancer type $c_2$ given that the patient has a cancer.

This may be repeated and the various probabilities compared to give the desired confidence that the patient has any particular cancer. In some embodiments the method further comprises concluding the patient has $c_1$ if $P(c_1)$ is higher than $P(c_2)$, $P(c_3)$, $P(c_4)$, ..., $P(c_x)$, where $P(c_2)$ through $P(c_x)$ represent the calculated probabilities of each cancer *(e.g.,* major cancers such as those listed in Tables 3 & 4) other than $c_1$.

It will often be useful to know what particular cancer is present. Thus one aspect of the invention provides a method of diagnosing cancer comprising:

(1) determining the mutational status of a first panel of genes;
(2) determining the mutational status of a second panel of genes; and
(3) calculating a likelihood $P(c_1)$ said patient has a particular cancer $c_1$ using the formula:

$$P(c_1|g_1, g_2, ..., g_n) = P_0(c_1) \; \Pi_i \; M(g_i| \; c_1) \; / \; \Sigma_t \; P_0(t) \; \Pi_i \; M(g_i|t)$$

wherein the product is taken over all genes in said second panel mutated in the sample *(i=1,2,...,n)*, the sum is taken over all cancer types *t, $M(g| \; c_1)$* is the frequency of somatic mutations in gene g from said second panel in cancer type $c_1$, and $P_0(c_1)$ is the *a priori* probability of cancer type $c_1$ given that the patient has a cancer.

As mentioned above, screening the five genes in Table 4 can detect nearly 95% of solid tumor types and the genes in Tables 2 & 3 can classify the cancer. Thus in some embodiments the presence of a mutation in any one of the genes listed in Table 4 is used as a pan-cancer screen to determine for which patients additional analysis should be done on a panel comprising at least one of the genes listed in Table 2 or 3. In some embodiments a mutation in any one of the genes in the first panel indicates the patient has cancer and application of the second panel classifies which type.

In some circumstances somatic mutations are the most informative mutations *(e.g.,* as in Example 1). In such cases one may determine the mutational status of the panel genes in both germline and somatic tissue to confirm that the mutation detected in the mutation screen is in fact somatic. In some embodiments this may be done with a single patient blood sample since germline mutational status may be determined from circulating blood cells while the somatic mutational analysis can be done with, *e.g.,* circulating tumor cells, exosomes derived from tumor cells, or circulating nucleic acids derived from tumor cells.

Calculating a patient's likelihood of having a particular cancer can be useful in various clinical settings. For example, if the calculated probability of the patient having a particular cancer is high enough one may diagnose the particular cancer, prescribe a treatment for the specific cancer, etc. If the patient is at particularly high risk of a specific cancer *(e.g.,* BRCA mutation carrier), then even a lower calculated likelihood of breast or ovarian cancer might be sufficient to make a diagnosis. A high likelihood of a particular cancer may alternatively prompt the doctor to recommend, prescribe, order, or perform an additional test *(e.g.,* biopsy, MRI, CT scan, digital rectal exam, mammography, etc.) to confirm the cancer.

Thus in aspects comprising calculating the likelihood of cancer $c_1$, some embodiments further comprise recommending, prescribing, ordering, or performing a test to confirm the presence of cancer $c_1$. In some embodiments the test is prescribed, ordered, recommended, or performed if the calculated likelihood exceeds some threshold value. In aspects comprising calculating the likelihood of cancer $c_1$ and the likelihood of cancer $c_2$, some embodiments further comprise recommending, prescribing, ordering, or performing a test to confirm the presence of cancer $c_1$ in said patient if the calculated likelihood said patient has said cancer $c_1$ is higher than the calculated likelihood said patient has cancer $c_2$. In some embodiments the test is prescribed, ordered, recommended, or performed if the calculated likelihood of $c_1$ exceeds that of $c_2$ and also exceeds some threshold value.

As used herein, a "panel of genes" is a plurality of genes. In some embodiments the panel consists of between 2 and 500, between 3 and 500, between 4 and 500, between 5 and 500, between 6 and 500, between 7 and 500, between 8 and 500, between 9 and 500, between 10 and 500, between 11 and 500, between 12 and 500, between 13 and 500, between 14 and 500, between 15 and 500, between 16 and 500, between 17 and 500, between 18 and 500, between 19 and 500, between 20 and 500, between 25 and 500, between 30 and 500, between 35 and 500, between 40 and 500, between 45 and 500, between 50 and 500, between 55 and 500, between 60 and 500, between 65 and 500, between 70 and 500, between 75 and 500, between 80 and 500, between 85 and 500, between 90 and 500, between 95 and 500, between 100 and 500, between 2 and 400, between 2 and 350, between 2 and 300, between 2 and 250, between 2 and 200, between 2 and 150, between 2 and 100, between 2 and 90, between 2 and 80, between 2 and 70, between 2 and 60, between 2 and 50, between 2 and 45, between 2 and 40, between 2 and 35, between 2 and 30, between 2 and 25, between 2 and 20, between 2 and 19, between 2 and 18, between 2 and 17, between 2 and 16, between 2 and 15, between 2 and 14, between 2 and 13, between 2 and 12, between 2 and 11, between 2 and 10, between 2 and 9, between 2 and 8, between 2 and 7, between 2 and 6, between 2 and 5, between 2 and 4, or between 2 and 3 genes and comprises at least one of the gene listed in Table 1 or a subset of the genes in Table 1. As used in the context of ranges, "between" includes the end of the range *(i.e.,* "between 2 and 500" includes both 2 and 500).

In some embodiments of the invention the panel comprises genes listed in Table 1 below:

**Table 1**

| Gene Abbrev. | Entrez GeneID | Gene Abbrev. | Entrez GeneID | Gene Abbrev. | Entrez GeneID |
|---|---|---|---|---|---|
| *AIM1* | 202 | *IDH1* | 3417 | *PMS1* | 5378 |
| *APC* | 324 | *KIT* | 3815 | *PMS2* | 5395 |
| *ATM* | 472 | *KRAS* | 3845 | *P TEN* | 5728 |
| *BRAF* | 673 | *HRAS* | 3265 | *RB1* | 5925 |
| *BRCA1* | 672 | *NRAS* | 4893 | *RET* | 5979 |
| *BRCA2* | 675 | *MAP2K4* | 6416 | *SMAD4* | 4089 |
| *CDKN2A* | 1029 | *MET* | 4233 | *SMO* | 6608 |
| *CD95 (aka FAS)* | 355 | *MLH1* | 4292 | *STK11* | 6794 |
| *CTNNB1* | 1499 | *MSH2* | 4436 | *TAF1L* | 138474 |
| *EGFR* | 1956 | *NF1* | 4763 | *TGFBR2* | 7048 |
| *FBN2* | 2201 | *NF2* | 4771 | *TNN* | 63923 |
| *FBXW7* | 55294 | *PIK3 CA* | 5290 | *TP53* | 7157 |
| *FLJ13479 (aka ZNF668)* | 79759 | *PIK3R1* | 5295 | *TRRAP* | 8295 |
| *FGFR3* | 2261 | *PRKDC* | 5591 | *VHL* | 7428 |

In some embodiments the panel comprises subsets (*e.g.*, at least 2, 3, 4, 5, 6, 7, 8, 9, 10, 15, 20, 25, 30, 35, 40 or more) of the genes in Table 1. In some embodiments the panel comprises *APC, EGFR, KRAS, PTEN,* and *TP53.* In some embodiments the panel comprises *AIM1, APC, CDKN2A, EGFR, FBN2, FBXW7, FLJ13479, IDH1, KRAS, PIK3CA, PIK3R1, PTEN, RB1, SMAD4, TGFBR2, TNN,* and *TP53.* In some embodiments the panel comprises *APC, ATM, BRAF, BRCA1, BRCA2, CDKN2A, CTNNB1, EGFR, FBXW7, FGFR3, KIT, KRAS, HRAS, NRAS, MAP2K4, MET, MLH1, MSH2, MSH6, NF1, NF2, PIK3CA, PRKDC, PTEN, RBI , RET, SMAD4, SMO, STK11, TAF1L, TP53, TRRAP,* and *VHL.* In some embodiments the panel comprises the genes listed in Table 4. In some embodiments the panel comprises the genes listed in Table 3. In some embodiments the panel comprises the genes listed in Table 1.

Mutations useful in the methods of the invention include missense mutations, deletions, insertions, frameshifts, copy number variations, and loss of heterozygosity. Deleterious mutations (i.e., mutations that reduce or abolish gene and/or protein function) are particularly relevant in the context of tumor suppressors (e.g., *APC, TP53, PTEN).* Activating mutations (i.e., mutations that increase gene and/or protein function) are particularly relevant in the context of oncogenes (e.g., *KRAS, EGFR).* Those skilled in the art are familiar with various deleterious and activating mutations for the genes listed in Tables 1, 3, and 4 (e.g., codons 12 and 13 in KRAS). Skilled artisans are also familiar with various techniques for determining whether a particular mutation is in fact deleterious or activating. For example, frameshift mutations resulting in early truncation of a tumor suppressor gene are generally expected to be deleterious. Table 7 includes mutations found in some of the genes listed in Table 1. Those skilled in the art are familiar with various resources and databases cataloguing mutations in the genes listed in Table 1. For example, the COSMIC [Catalogue of Somatic Mutations in Cancer] database currently contains over 26,000 entries for these genes. Those skilled in the art will be able to use these entries in the methods of the invention for detecting and classifying cancer.

As used herein, determining the "mutational status" of a gene means determining at least one of the following: (a) whether the gene (or any of its products) harbors a sequence mutation (including point mutations, deletions, insertions, copy number variants, etc.), (b) the prevalence of such mutations in a sample, or (c) whether such a sequence mutation is activating or inactivating. Thus a particular mutational status includes, but is not limited to, the presence or absence of a mutation, a relatively high or relatively low prevalence of a mutation, an inactivating mutation, an activating mutation, etc. In some embodiments determining the mutational status of a gene comprises assaying some marker whose status itself is correlated with the mutational status of the gene of interest. Determining the mutational status of a panel of genes means determining the mutational status of each gene in the panel.

Mutational status of a gene may be determined by any of several techniques familiar to those skilled in the art. Exemplary techniques include resequencing (either of selected regions of the gene or of the entire gene), allele-specific amplification *(e.g.,* TaqMan™ using mutant allele-specific probes), microarray analysis *(e.g.,* arrays for CNV or arrays containing mutant allele-specific probes), etc. In some embodiments of the invention the method comprises physically amplifying and/or isolating nucleic acid of a panel of genes from a sample obtained from a patient. As used herein, "amplifying a nucleic acid" and "isolating nucleic acid" have their conventional meanings in the art. Thus in some embodiments the method further comprises amplifying nucleic acid of a panel of genes *(e.g.,* comprising the genes listed in Table 3) from a sample obtained from a patient, determining the mutational status of each gene in the panel, and calculating the likelihood of a particular cancer as discussed above and below.

"Sample" as used herein refers to any biological specimen, including any tissue or fluid, that can be obtained from, or derived from a specimen obtained from, a human subject. Such samples include but are not limited to healthy or tumor tissue, bodily fluids *(e.g.,* blood), waste matter *(e.g.,* urine, stool), etc. "Bodily fluid sample" as used herein means any fluid that can be extracted or collected from a human body. In some embodiments of each aspect of the invention the bodily fluid sample is blood or a blood derivative. Examples of blood derivatives include, but are not limited to, plasma and serum. In some embodiments the bodily fluid sample is urine, stool, pleural effusion, lacrimal effusion, saliva, sputum, etc. As used herein, "analyzing genes in a sample" refers to analyzing nucleic acids corresponding to those genes in a sample or any substance derived from that sample. For example, analyzing the *APC, EGFR, KRAS, PTEN* and *TP53* genes in blood includes analyzing PCR™ amplified portions of these genes in a patient blood sample (including plasma or serum), or in DNA or RNA isolated (i.e., derived) from such a sample. In some embodiments such a nucleic acid is chosen from the group consisting of genomic DNA (including PCR™ amplified copies of genomic DNA), mRNA, cDNA, and a portion of any of these.

The cancer screening and classification methods of the inventions will often involve analyzing nucleic acids from bodily fluids since these are often the least invasive samples to obtain from patients. For example, the method of the invention may involve isolating nucleic acids from circulating tumor cells from the blood. This may involve capturing circulating tumor cells (e.g., using tumor-specific capture antibodies) and subsequent analysis of the DNA or RNA contained in the cell. Alternatively, the methods of the invention may isolate and analyze nucleic acids that float freely in the bodily fluid. As discussed in more detail below, the methods of the invention may also isolate nucleic acids from extracellular vesicles found in the bodily fluid sample.

Mutations in some of genes are associated with particular cancer types. As used herein, "cancer type" and "type of cancer" mean a cancer in or originating from a particular tissue or organ and/or a cancer with a particular molecular or clinical feature. Often, the specificity of the "cancer type" varies with the application, including tissue type *(e.g.,* squamous versus cuboidal), organ type *(e.g.,* breast versus lung), and clinical subtype *(e.g.,* triple-negative breast cancer). Thus another aspect of the invention provides a method of classifying cancer comprising isolating nucleic acids corresponding to a panel of genes from a sample obtained from a patient and determining the mutational status of each such nucleic acid, wherein a particular mutational status in particular genes in the panel indicates the patient has a particular cancer. Those skilled in the art will appreciate that methods according to this aspect may simultaneously detect and classify cancer. In some embodiments the panel comprises the *AIM1, APC, CDKN2A, EGFR, FBN2, FBXW7, FLJ13479, IDH1, KRAS, PIK3CA, PIK3R1, PTEN, RB1, SMAD4, TGFBR2, TNN,* and *TP53* genes or a subset (e.g., at least 3, 4, 5, 6, 7, 8, 9, 10 or 15 or more) thereof. In other embodiments the panel comprises the *APC, ATM, BRAF, BRCA1, BRCA2, CDKN2A, CTNNB1, EGFR, FBXW7, FGFR3, KIT, KRAS, HRAS, NRAS, MAP2K4, MET, MLH1, MSH2, MSH6, NF1, NF2, PIK3CA, PRKDC, PTEN, RB1, RET, SMAD4, SMO, STK11, TAF1L, TP53, TRRAP,* and *VHL* genes or a subset *(e.g.,* at least 3, 4, 5, 6, 7, 8, 9, 10, 15, 20, 25, or 30 or more) thereof. In still other embodiments the panel comprises the *AIM1, APC, ATM, BRAF, BRCA1, BRCA2, CDKN2A, CD95, CTNNB1, EGFR, FBN2, FBXW7, FLJ13479, FGFR3, IDH1, KIT, KRAS, HRAS, NRAS, MAP2K4, MET, MLH1, MLH2, MSH1, MSH2, NF1, NF2, PIK3CA, PIK3R1, PRKDC, PTEN, PMS1, PMS2, RB1, RET, SMAD4, SMO, STK11, TAF1L, TGFBR2, TNN, TP53, TRRAP,* and *VHL* genes or a subset (e.g., at least 3, 4, 5, 6, 7, 8, 9, 10, 15, 20, 25, 30, 35, 40 or more) thereof.

As used herein, "classifying a cancer" and "cancer classification" refer to determining one or more clinically-relevant features of a cancer. Thus "classifying a cancer" includes, but is not limited to: (i) determining the tissue type or organ of origin of the cancer (e.g., cancer type); (ii) determining clinical subtype of cancer *(e.g.,* EGFR amplified); (iii) evaluating metastatic potential, potential to metastasize to specific organs, risk of recurrence, and/or course of the tumor; (iv) evaluating tumor stage; (v) determining patient prognosis in the absence of treatment of the cancer; (vi) determining prognosis of patient response *(e.g.,* tumor shrinkage or progression-free survival) to treatment *(e.g.,* chemotherapy, radiation therapy, surgery to excise tumor, etc.); (vii) diagnosis of actual patient response to current and/or past treatment; (viii) determining a preferred course of treatment for the patient; (ix) prognosis for patient relapse after treatment (either treatment in general or some particular treatment); (x) prognosis of patient life expectancy *(e.g.,* prognosis for overall survival), *etc.* The methods of the invention are particularly suited to determining tumor origin.

The cancer screening and cancer classification aspects of the invention may also be combined to provide a method for diagnosing specific cancer types. This will often involve screening a patient for the presence of cancer generally and, if it is present, classifying the cancer. Thus this aspect of the invention provides a method of diagnosing cancer comprising (1) isolating nucleic acids corresponding to a first panel of genes from a sample obtained from a patient; and (2) determining the mutational status of each nucleic acid corresponding to a gene in the first panel, wherein a particular mutational status in particular genes in the first panel indicates the patient has cancer; (3) isolating nucleic acids corresponding to a second panel of genes from the sample; (4) determining the mutational status of each nucleic acid corresponding to a gene in the second panel, wherein a particular mutational status in particular genes in the second panel indicates the patient has a particular cancer type. As used herein, "cancer type" refers to tissue, tissue type or organ of origin for a cancer.

In some embodiments the isolating steps (1) and (3) are performed sequentially. This allows for a relatively less expensive, quicker initial assessment of the general presence of cancer which can, if necessary, be followed with further analysis of more genes to determine cancer type. Alternatively, in other embodiments the isolating steps (1) and (3) are done at the same time-*i.e.*, they are in essence collapsed into a single step that isolates and/or analyzes nucleic acids from both panels simultaneously. Isolation and analysis may be performed on the same patient sample or on different samples.

In some embodiments the first panel comprises the *APC, EGFR, KRAS, PTEN,* and *TP53* genes and the second panel comprises the *AIM1, APC, ATM, BRAF, BRCA1, BRCA2, CDKN2A, CD95, CTNNB1, EGFR, FBN2, FBXW7, FLJ13479, FGFR3, IDH1, KIT, KRAS, HRAS, NRAS, MAP2K4, MET, MLH1, MLH2, MSH1, MSH2, NF1, NF2, PIK3CA, PIK3R 1, PRKDC, PTEN, PMS1, PMS2, RB1, RET, SMAD4, SMO, STK11, TAF1L, TGFBR2, TNN, TP53, TRRAP,* and *VHL* genes or subsets thereof (*e.g., AIM1, APC, CDKN2A, EGFR, FBN2, FBXW7, FLJ13479, IDH1, KRAS, PIK3CA, PIK3R1, PTEN, RB1, SMAD4, TGFBR2, TNN,* and *TP53* or *APC, ATM, BRAF, BRCA1, BRCA2, CDKN2A, CTNNB1, EGFR, FBXW7, FGFR3, KIT, KRAS, HRAS, NRAS, MAP2K4, MET, MLH1, MSH2, MSH6, NF1, NF2, PIK3CA, PRKDC, PTEN, RB1, RET, SMAD4, SMO, STK11, TAF1L, TP53, TRRAP,* and *VHL*).

Knowing that a patient has cancer can be valuable in various clinical settings beyond diagnosis. Thus other aspects of the invention provide methods of detecting cancer in a patient identified as being at heightened risk of having or developing cancer, methods of monitoring cancer therapy (e.g., for recurrence or progression), methods of determining whether a patient is a candidate for biopsy or other further testing, methods of determining drug response, etc. These methods will generally comprise isolating nucleic acids corresponding to a panel of genes from a patient sample and determining the mutational status of each such nucleic acid, wherein a particular mutational status in particular genes in the panel will indicate some particular clinical feature (*e.g.*, desirability of biopsy, desirability of a particular treatment, etc.). For example, a panel of genes comprising *KRAS* may be assayed to determine that a patient has colon cancer, with knowledge of an activating mutation in *KRAS* further indicating a decreased likelihood of response to anti-EGFR therapy.

Thus one aspect of the invention provides a method of screening for cancer in a patient comprising identifying a patient at risk of having, or in need of screening for, cancer and determining the mutational status of a panel of genes in a sample obtained from the patient, wherein a particular mutational status in the sample indicates the presence of cancer. Patients may be identified as at risk of having, or in need of screening for, cancer in a variety of ways and based on numerous clinical and/or molecular characteristics. One class of patients at risk of having cancer and in need of screening is those patients known to carry a germline deleterious mutation in a tumor suppressor gene. Examples include, but are not limited to, *BRCA1* (breast or ovarian), *BRCA2* (breast or ovarian), *PTEN* (glioma), *p16* (melanoma), *MLH1* (colorectal), *MSH6* (colorectal), *APC* (colorectal), *MYH* (colorectal), etc. In such patients, cancer-type specificity is often less crucial since, for example, a *BRCA1*-mutant patient whose mutational status in a panel of predictive genes (*e.g., APC, EGFR, KRAS, PTEN,* and *TP53*) indicates cancer would be expected have breast or ovarian cancer rather than some other type of cancer. The relatively non-invasive nature of serum detection (*i.e.,* simple blood draw) makes such widespread screening attractive and practical.

Thus in some embodiments the invention provides a method of detecting cancer comprising identifying a patient having a mutation in a gene selected from the group consisting of *BRCA1, BRCA2, PTEN, p16, MLH1, MSH6, APC,* and *MYH;* and determining the mutational status of a panel of genes in a sample obtained from the patient; wherein a particular mutational status indicates the presence of cancer. In some such embodiments the method further comprises additional tests to determine/confirm which type of cancer is present.

Another aspect of the invention provides a method of detecting recurrence in a cancer patient comprising determining the mutational status of a panel of genes in a sample obtained from the patient, wherein a particular mutational status indicates recurrence. Because it is difficult to remove or kill all cancerous cells, one of the main challenges in cancer treatment is making sure a cancer removed by surgery and/or treated with drugs has not returned. Thus this aspect of the invention is particularly useful in monitoring cancer patients following treatment. Much like the at-risk patients discussed above, cancer-type specificity is often not crucial: If a lung cancer patient is found to have a particular mutational status in his serum several months or years after treatment, then the new cancer is likely to be a return of the former lung cancer. As above, in some embodiments further testing (*e.g.,* imaging) to confirm the type of cancer or to characterize the cancer (*e.g.,* stage) is encompassed by the invention. In some embodiments mutational status is measured soon after treatment (*e.g.,* to determine a post-treatment baseline) and then monitored at regular intervals there after in order to catch any significant change (*e.g.,* from this baseline).

Yet another promising way in which the invention may be used clinically is to identify patients who need further testing to confirm the existence, location, and/or character of a cancer. Biopsies, for example, are generally quite invasive, involving substantial discomfort and risk *(e.g.,* infection). Imaging tests *(e.g.,* MRI, CT scan, etc.) are generally less invasive, but are very expensive and some *a priori* idea of the location of a tumor is generally needed. By indicating which patients are likely to have cancer in a particular organ or tissue, the methods of the present invention may be used to identify patients who are good candidates for biopsy or imaging. For example, the invention provides a method of diagnosing cancer comprising isolating nucleic acids corresponding to a panel of genes from a sample obtained from a patient; determining the mutational status of each such nucleic acid, wherein a particular mutational status in particular genes in the first panel indicates the patient has a particular cancer; and recommending, prescribing or performing further testing to confirm the presence, location or character of the cancer. In some embodiments the further testing comprises a biopsy or an imaging test. In some embodiments, especially if the genetic screen indicates cancer in a large organ like the lung, further testing may involve an imaging test to better pinpoint the location of any mass and then biopsy to further analyze the mass *(e.g.,* to confirm malignancy). In the case of patients already identified as at-risk for particular cancers *(e.g., BRCA* mutation carriers), a simple pan-cancer screen according to the present invention may give the information necessary to propmt further testing of the at-risk area *(e.g.,* breasts or ovaries).

Nucleic acids *(e.g.,* mRNA) for analysis according to the present invention may come from any suitable source, especially those likely to be enriched for tumor nucleic acids. One example may be tumor tissue itself *(e.g.,* unknown metastasis for which origin is to be determined). In another example, the blood (or serum or plasma) of a patient may be treated to isolate mRNA or DNA for mutation analysis since such body fluids carry circulating mRNA and DNA. This nucleic acid may come from circulating tumor cells or it may be free circulating nucleic acid. Techniques for isolating and analyzing nucleic acids from blood and blood derivatives are known to those skilled in the art. *See, e.g.,* U.S. Pat. No. 7,442,507. Thus in some embodiments of the invention the sample is a bodily fluid *(e.g.,* blood, pleural fluid, urine, etc.). In some embodiments the bodily fluid is blood. In some embodiments the sample is a blood derivative such as serum or plasma.

An additional source of nucleic acids is small extracellular vesicles, including exosomes, which are abundant in the blood (and serum and plasma) of cancer patients due to increased production by tumor cells. This is especially true of epithelial cancers *(e.g.,* those of the lung, colon, breast, prostate, ovaries, endometrium, etc). Exosomes carry important biomolecules on their surface *(e.g.,* protein) and within their interior *(e.g.,* mRNA). Because exosomes are often derived from tumor cells, the biomolecules they carry can provide valuable information regarding the tumor cells from which they are derived. Thus, circulating exosomes, by generally yielding a relatively high concentration of tumor-derived mRNA, can provide an enriched snapshot or non-invasive "virtual biopsy" of tumor cells. This is especially helpful in general cancer screening, where minimal invasiveness is particularly advantageous. mRNA from exosomes may be isolated and analyzed to determine the mutational status of genes.

Thus in some embodiments of the invention nucleic acids are isolated from exosomes obtained from a patient blood (or blood derivative) sample. Several techniques for isolating nucleic acids from exosomes and for isolating exosomes themselves are known in the art. *See, e.g.,* U.S. Pat. No. 7,198,923. Examples include differential centrifugation, immunoseparation, bead-assisted centrifugation, fluorescence-assisted cell sorting (FACS), affinity chromatography, etc. At times it will be desirable to differentiate tumor-derived exosomes from exosomes derived from some other cell, especially since normal immune cells in the blood release exosomes. This can be done, *e.g.,* by FACS or immunocentrifugation using a surface marker specific for cancer or a marker specific for non-immune cells (e.g., epithelial membrane antigen [EMA] or EpCAM).

Other information may be combined with mutational status in some aspects of the invention. For example, expression levels of certain genes often differ between cancer and non-cancer and among different cancer types and subtypes. Thus some embodiments provide methods as described below further comprising determining the expression level of a gene, wherein a particular mutational status and a high expression level indicate cancer, a particular cancer type, etc. Examples of such genes whose expression level is often informative include, but are not limited to, *EGFR, HER2,* PSA, CA125, CEA, etc. Determining the expression level of a gene can include determining the amount of mRNA and/or protein products of the gene. In some embodiments the level (including presence, absence, or qualitative amount) of a marker is used not so much to indicate cancer or cancer type, but instead simply to indicate tissue or organ type from which the nucleic acid *(e.g.,* by way of an exosome) is derived. Examples include EpCAM, 34βE12, Ae1/3, AFP, B72.3, CA-125, Calictonin, Calretinin, CAM5.2, CD10, CD15, CD56, CEA, Chromogranin, CK19, CK5/6, cytokeratin 20, cytokeratin 7, EMA, GCDFP-15, HBME-1, HepPar1, HER2, Leu, Leu7, M1, Mesothelin, Mucicarmine, NCAM, PSA, PSAP, PSMA, RCC, Synaptophysin, Thyroglobulin, UroplakinIII, Villin, Vimentin, etc.

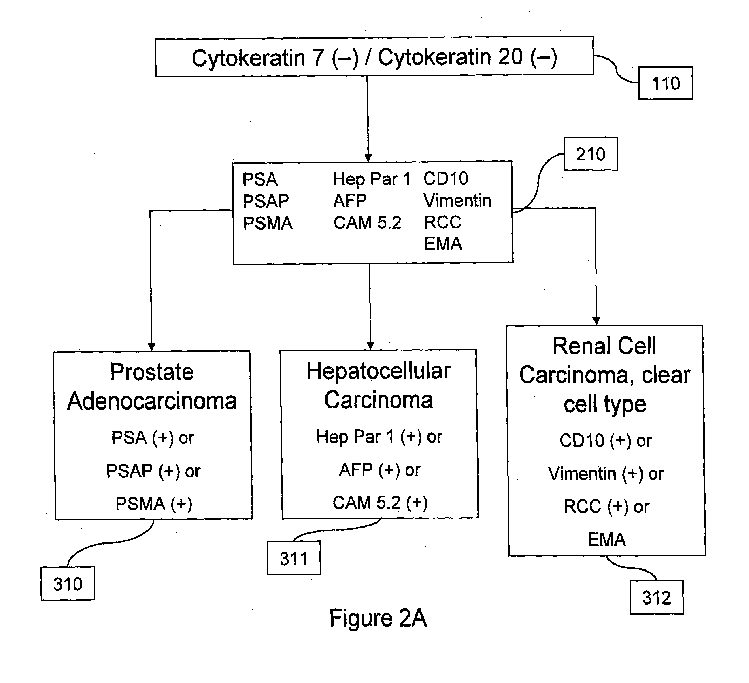

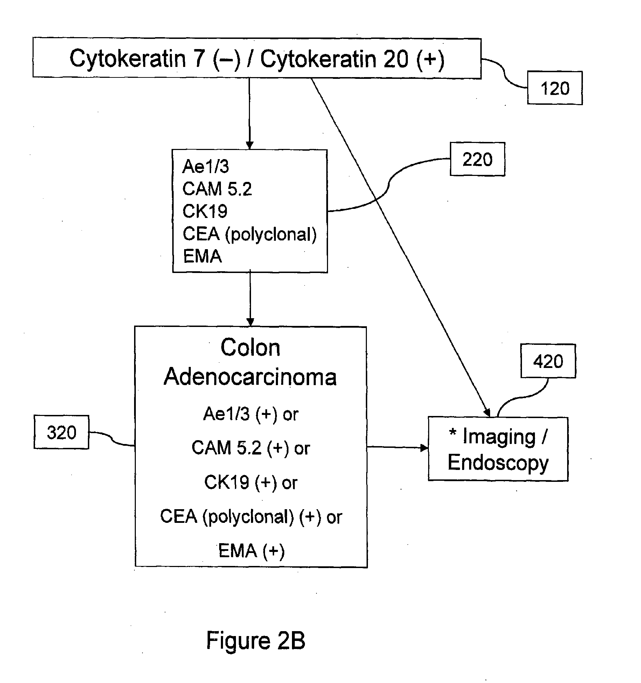

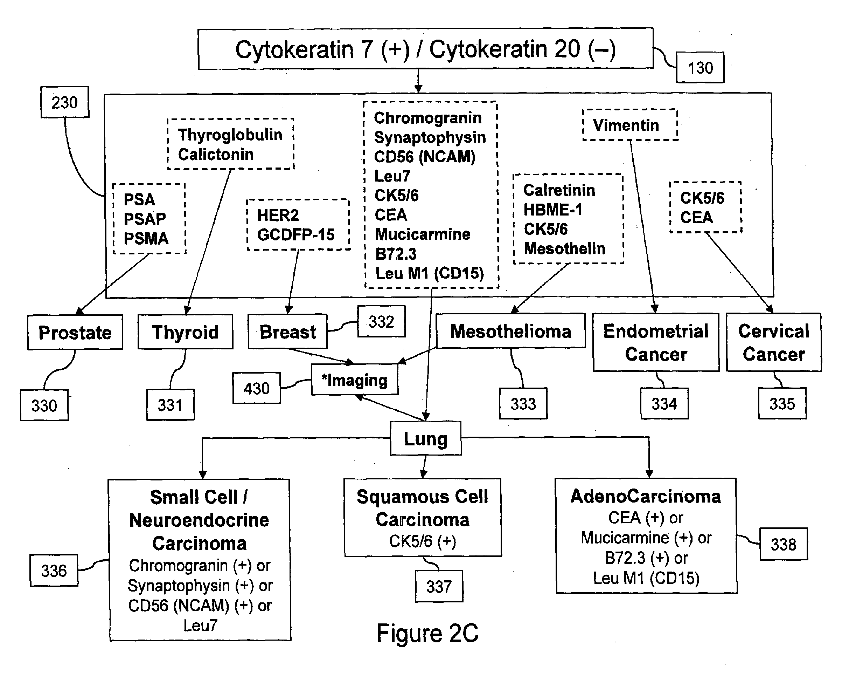

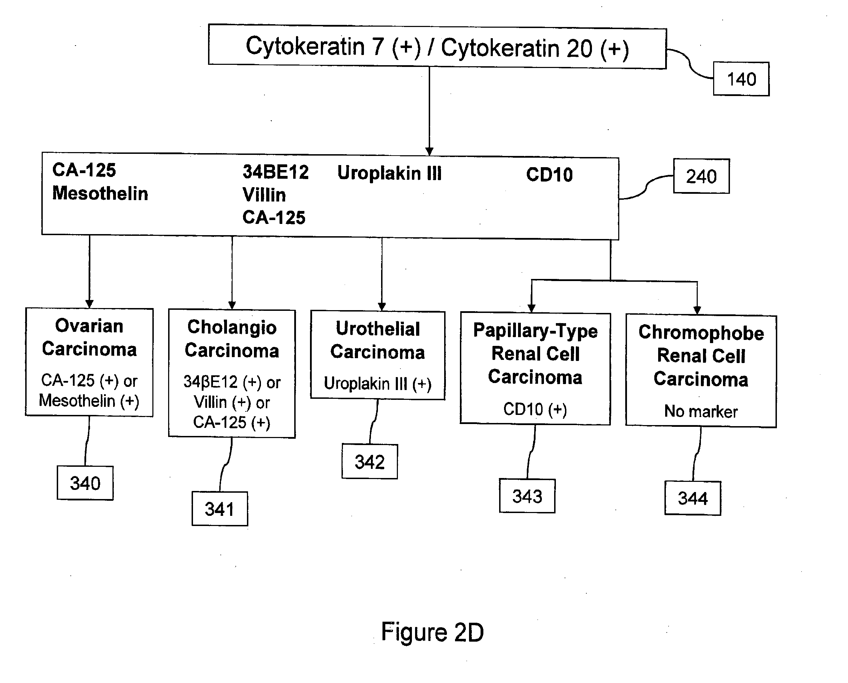

In some embodiments the panel of tissue markers comprises two or more markers shown in FIG.2, wherein the presence or absence (or abnormal status) of specific markers indicates, according to the flowcharts in FIG.2, the patient has cancer of a specific type.

In further embodiments the status of individual markers in the panel is tested in a certain order in order to narrow down which specific cancer type is present. One example is illustrated in FIG.2A-2D. Specifically, when a particular mutational status is found in a patient's sample, one may also test the sample for the status of cytokeratin 7 (CK7) and cytokeratin 20 (CK20) followed by various other markers. If both CK7 and CK20 are absent as in FIG.2A [110], then PSA, PSAP, PSMA, Hep Par 1, AFP, CAM 5.2, CD10, Vimentin, RCC, and EMA (or any combination thereof or any single marker) may be tested **[210]** to determine the specific organ/tissue of origin. If PSA, PSAP, and/or PSMA are found, then the cancer is prostate adenocarcinoma **[310]**. If Hep Par 1, AFP, and/or CAM 5.2 are present, then the cancer is hepatocellular carcinoma **[311]**. If CD10, Vimentin, RCC, and/or EMA are present, then the cancer is renal cell carcinoma (clear cell type) **[312]**.

If CK7 is absent and CK20 is present as in FIG.2B **[120]**, then Ae1/3, CAM 5.2, CK19, CEA (polyclonal), and EMA (or any combination thereof or any single marker) may be tested **[220]** to confirm that the cancer is colon adenocarcinoma. If any of these markers is found, then the cancer is colon adenocarcinoma **[320]**. Imaging and/or endoscopy may be performed **[420]** either in place of the additional marker tests **[320]** or as an additional confirmation.

If CK7 is present and CK20 is absent as in FIG.2C **[130]**, then PSA, PSAP, PSMA, Thyroglobulin, Calictonin, HER2, GCDFP-15, Chromogranin, Synaptophysin, CD56, (NCAM), Leu7, CK5/6, CEA, Mucicarmine, B72.3, Leu, M1, (CD15), Calretinin, HBME-1, Mesothelin and Vimentin (or any combination thereof or any single marker) may be tested **[230]** to determine the specific organ/tissue of origin. If PSA, PSAP, and/or PSMA are found, then the cancer is prostate cancer **[330]**. If Thyroglobulin and/or Calictonin are present, then the cancer is thyroid cancer **[331]**. If HER2 and/or GCDFP-15 are found, then the cancer is breast cancer **[332]**. If Chromogranin, Synaptophysin, CD56, (NCAM), and/or Leu7 are found, then the cancer is small cell/neuroendocrine carcinoma of the lung **[336]**. If CK5/6 is found, then the cancer may be squamous cell carcinoma of the lung **[337]** (diagnosis may be confirmed by imaging **[430]**). CEA, Mucicarmine, B72.3, and/or Leu M1 (CD15) are found, then the cancer may be adenocarcinoma of the lung **[338]** (diagnosis may be confirmed by imaging **[430]**). If Calretinin, HBME-1, CK5/6, and/or Mesothelin are found, then the cancer may be mesothelioma **[333]** (if the only marker found is CK5/6, imaging **[430]** may be necessary). If Vimentin is found, then the cancer is endometrial cancer **[334]**. If CK5/6 and/or CEA are found, then the cancer may be cervical cancer **[332]** (confirmation, e.g., by pap smear, may be necessary since these markers are also expressed by other CK7+/CK20- tissue types).

If CK7 and CK20 are both present as in FIG.2D **[140]**, then CA-125, Mesothelin, 34βE12, Villin, Uroplakin III, and/or CD10 (or any combination thereof or any single marker) may be tested **[240]** to determine the specific organ/tissue of origin. If CA-125 and/or Mesothelin are found, then the cancer may be ovarian carcinoma **[340]** (confirmation, e.g., by imaging, may be necessary since CA-125 is also expressed in other CK7+/CK20+ tissues). If 34βE12, Villin, and/or CA-125 are present, then the cancer may be cholangio carcinoma (bile duct cancer) **[341]** (confirmation, e.g., by imaging, may be necessary since CA-125 is also expressed in other CK7+/CK20+ tissues). If Uroplakin III is found, then the cancer is urothelial carcinoma **[342]**. If CD10 is found, then the cancer is papillary-type renal cell carcinoma **[343]**. If no marker is found, then the cancer may be chromophobe renal cell carcinoma **[344]** (diagnosis may be confirmed microscopically).

As mentioned above, some embodiments of the invention involve mutational analysis combined with more traditional diagnostic techniques. For example, physical examination (e.g., digital rectal exam for prostate cancer), imaging (e.g., mammography), and/or biopsy may be used to confirm a diagnosis indicated by mutational analysis according to the invention. Alternatively, such techniques may be combined with mutational analysis (and optionally exosome surface marker analysis) to yield a more comprehensive diagnosis. As an illustrative example, a mutational screen may indicate the presence of cancer in a patient and exosomes may be found to be CK7+/CK20-and have the marker CK5/6 associated with them. One may not be able to conclusively determine based solely on this information whether the cancer is squamous cell carcinoma of the lung, cervical cancer, or mesothelioma at some unknown organ (see FIG.2C). Thus, a physician may take the further step of imaging to pinpoint the location of the cancer (e.g., in or near the lung). The physician may further perform a biopsy to determine whether the cancer is squamous cell carcinoma of the lung or cancer of the mesothelial lining of the lung.

As used herein in the context of biomarkers and their expression, the "level" of something in a sample has its conventional meaning in the art. Determining a "level" herein includes quantitative determinations-e.g., mg/mL, fold change, etc. Determining a "level" herein also includes qualitative determinations-e.g., determining the presence or absence of a marker or determining whether the level of the marker is "high," "low" or even "present" relative to some index value.

In one embodiment, in determining the level of expression in accordance with the present invention the amount of expression is measured within one or more samples and compared to some index value. The index value may represent the average expression level of a marker in a plurality of training patients (e.g., both diseased and healthy patients). For example, a "cancer index value" can be generated from a plurality of training patients characterized as having cancer. A "cancer-free index value" can be generated from a plurality of training patients defined as not having cancer. Thus, a cancer index value of expression may represent the average level of expression in patients having cancer, whereas a cancer-free index value of expression may represent the average level of expression in patients not having cancer. Thus, when the level of expression is more similar to the cancer index value than to the cancer-free index value, then it can be concluded that the patient has or is likely to have cancer. On the other hand, if the level of expression is more similar to the cancer-free index value than to the cancer index value, then it can be concluded that the patient does not have or has no increased likelihood of having cancer.

The results of these and any other analyses according to the invention will often be communicated to physicians, genetic counselors and/or patients (or other interested parties such as researchers) in a transmittable form that can be communicated or transmitted to any of the above parties. Such a form can vary and can be tangible or intangible. The results can be embodied in descriptive statements, diagrams, photographs, charts, images or any other visual forms. For example, graphs showing mutational status information for various genes can be used in explaining the results. Diagrams showing such information for additional target gene(s) are also useful in indicating some testing results. The statements and visual forms can be recorded on a tangible medium such as papers, computer readable media such as floppy disks, compact disks, *etc.,* or on an intangible medium, *e.g.,* an electronic medium in the form of email or website on internet or intranet. In addition, results can also be recorded in a sound form and transmitted through any suitable medium, e.g., analog or digital cable lines, fiber optic cables, *etc.,* via telephone, facsimile, wireless mobile phone, internet phone and the like.

Thus, the information and data on a test result can be produced anywhere in the world and transmitted to a different location. As an illustrative example, when an assay is conducted outside the United States, the information and data on a test result may be generated, cast in a transmittable form as described above, and then imported into the United States. Accordingly, the present invention also encompasses a method for producing a transmittable form of information on at least mutational status for a panel of genes for at least one patient sample. The method comprises the steps of (1) determining mutational status as described above according to methods of the present invention; and (2) embodying the result of the determining step in a transmittable form. The transmittable form is the product of such a method. Thus the processing of physical samples may be temporally and physically separated from their analysis in the methods of the invention. Indeed, mutational status may be determined in a blood sample for some other purpose and the stored mutational data from an earlier assay may be applied to the methods of the invention in diagnosing cancer.

Techniques for analyzing mutational status or expression (indeed any data obtained according to the invention) will often be implemented using hardware, software or a combination thereof in one or more computer systems or other processing systems capable of effectuating such analysis. The computer-based analysis function can be implemented in any suitable language and/or browsers. For example, it may be implemented with C language and preferably using object-oriented high-level programming languages such as Visual Basic, SmallTalk, C++, and the like. The application can be written to suit environments such as the Microsoft Windows™ environment including Windows™ 98, Windows™ 2000, Windows™ NT, and the like. In addition, the application can also be written for the MacIntosh™, SUN™, UNIX or LINUX environment. In addition, the functional steps can also be implemented using a universal or platform-independent programming language. Examples of such multi-platform programming languages include, but are not limited to, hypertext markup language (HTML), JAVA™, JavaScript™, Flash programming language, common gateway interface/structured query language (CGI/SQL), practical extraction report language (PERL), AppleScript™ and other system script languages, programming language/structured query language (PL/SQL), and the like. Java™- or JavaScript™-enabled browsers such as HotJava™, Microsoft™ Explorer™, or Netscape™ can be used. When active content web pages are used, they may include Java™ applets or ActiveX™ controls or other active content technologies.

The analysis function can also be embodied in computer program products and used in the systems described above or other computer- or internet-based systems. Accordingly, another aspect of the present invention relates to a computer program product comprising a computer-usable medium having computer-readable program codes or instructions embodied thereon for enabling a processor to carry out gene status analysis. These computer program instructions may be loaded onto a computer or other programmable apparatus to produce a machine, such that the instructions which execute on the computer or other programmable apparatus create means for implementing the functions or steps described above. These computer program instructions may also be stored in a computer-readable memory or medium that can direct a computer or other programmable apparatus to function in a particular manner, such that the instructions stored in the computer-readable memory or medium produce an article of manufacture including instruction means which implement the analysis. The computer program instructions may also be loaded onto a computer or other programmable apparatus to cause a series of operational steps to be performed on the computer or other programmable apparatus to produce a computer implemented process such that the instructions which execute on the computer or other programmable apparatus provide steps for implementing the functions or steps described above.

Thus in some embodiments the invention provides a method comprising: accessing mutational status information derived from a patient sample and stored in a computer-readable medium; querying this information to determine whether the patient has a particular mutational status for a panel of genes; calculating the likelihood of the patient having a particular cancer type based on the mutational status of the panel; outputting [or displaying] the likelihood of the patient having a particular cancer type based on the mutational status of the panel. A similar computer-implemented diagnostic method may use a panel of genes to indicate likelihood of the presence of cancer generally. Yet another method may combine the pan-cancer screen and the caner type- specific screen described above. For example, one embodiment provides a method comprising: accessing mutational status information on a first panel of genes derived from a patient sample and stored in a computer-readable medium; querying this information to determine whether the patient has a particular mutational status for the first panel; calculating the likelihood of the patient having cancer based on the mutational status of the first panel; accessing mutational status information on a second panel of genes derived from a patient sample and stored in a computer-readable medium; querying this information to determine whether the patient has a particular mutational status for the second panel; calculating the likelihood of the patient having a particular cancer based on the mutational status of the second panel; outputting [or displaying] the likelihood of the patient having a particular cancer type based on the mutational status of the second panel. One may optionally also output [or display] the likelihood of the patient having cancer generally, either before analyzing the mutational status information for the second panel or together with the output of the likelihood of the patient having a particular cancer type. Some embodiments further comprise displaying the mutational status information.

As used herein in the context of computer-implemented embodiments of the invention, "displaying" means communicating any information by any sensory means. Examples include, but are not limited to, visual displays, *e.g.,* on a computer screen or on a sheet of paper printed at the command of the computer, and auditory displays, *e.g.,* computer generated or recorded auditory expression of a patient's genotype.

The practice of the present invention may also employ conventional biology methods, software and systems. Computer software products of the invention typically include computer readable media having computer-executable instructions for performing the logic steps of the method of the invention. Suitable computer readable medium include floppy disk, CD-ROM/DVD/DVD-ROM, hard-disk drive, flash memory, ROM/RAM, magnetic tapes and etc. Basic computational biology methods are described in, for example, Setubal et al., INTRODUCTION TO COMPUTATIONAL BIOLOGY METHODS (PWS Publishing Company, Boston, 1997); Salzberg et al. (Ed.), COMPUTATIONAL METHODS IN MOLECULAR BIOLOGY, (Elsevier, Amsterdam, 1998); Rashidi & Buehler, BIOINFORMATICS BASICS: APPLICATION IN BIOLOGICAL SCIENCE AND MEDICINE (CRC Press, London, 2000); and Ouelette & Bzevanis, BIOINFORMATICS: A PRACTICAL GUIDE FOR ANALYSIS OF GENE AND PROTEINS (Wiley & Sons, Inc., 2nd ed., 2001); *see also,* U.S. Pat. No. 6,420,108.

The present invention may also make use of various computer program products and software for a variety of purposes, such as probe design, management of data, analysis, and instrument operation. *See* U.S. Pat. Nos. 5,593,839; 5,795,716; 5,733,729; 5,974,164; 6,066,454; 6,090,555; 6,185,561; 6,188,783; 6,223,127; 6,229,911 and 6,308,170. Additionally, the present invention may have embodiments that include methods for providing genetic information over networks such as the Internet as shown in U.S. Ser. Nos. 10/197,621 (U.S. Pub. No. 20030097222); 10/063,559 (U.S. Pub. No. 20020183936), 10/065,856 (U.S. Pub. No. 20030100995); 10/065,868 (U.S. Pub. No. 20030120432); 10/423,403 (U.S. Pub. No. 20040049354).

Another aspect of the invention provides microarrays and kits (including a microarray kit) for practicing the methods of the invention. The kit may include a carrier for its various components. The carrier can be a container or support, in the form of, *e.g.,* bag, box, tube, rack, and is optionally compartmentalized. The carrier may define an enclosed confinement for safety purposes during shipment and storage.

Microarrays and kits (including microarray kits) of the invention may comprise reagents for determining the mutational status of a panel of genes consisting of between 5 and 5,000 genes and comprising at least one gene chosen from the group consisting of: *AIM1, APC, ATM, BRAF, BRCA1, BRCA2, CDKN2A, CD95, CTNNB1, EGFR, FBN2, FBXW7, FLJ13479, FGFR3, IDH1, KIT, KRAS, HRAS, NRAS, MAP2K4, MET, MLH1, MLH2, MSH1, MSH2, NF1, NF2, PIK3CA, PIK3R1, PRKDC, PTEN, PMS1, PMS2, RB1, RET, SMAD4, SMO, STK11, TAF1L, TGFBR2, TNN, TP53, TRRAP,* and *VHL.* In some embodiments the panel comprises subsets of these genes, *e.g., APC, EGFR, KRAS, PTEN,* and *TP53;* or *AIM1, APC, CDKN2A, EGFR, FBN2, FBXW7, FLJ13479, IDH1, KRAS, PIK3CA, PIK3R1, PTEN, RB1, SMAD4, TGFBR2, TNN,* and *TP53;* or *APC, ATM, BRAF, BRCA1, BRCA2, CDKN2A, CTNNB1, EGFR, FBXW7, FGFR3, KIT, KRAS, HRAS, NRAS, MAP2K4, MET, MLH1, MSH2, MSH6, NF1, NF2, PIK3CA, PRKDC, PTEN, RB1, RET, SMAD4, SMO, STK11, TAF1L, TP53, TRRAP,* and *VHL.*

Those skilled in the art are familiar with various reagents that may be used for determining whether a particular gene harbors a mutation. For example, one may use oligonucleotide probes (e.g., probes specific for a mutant allele) and/or primers (e.g., PCR primers in RT-PCR reactions) to determine mutational status. In some embodiments the invention provides the use of such reagents for the manufacture of an invitro diagnostic kit.

Kits of the invention may further comprise reagents *(e.g.,* antibodies) for assessing the status *(e.g.,* presence, absence, level) of various additional markers, *e.g.,* those given in FIG.2. These reagents and optionally included apparatuses may be useful in enzyme-linked immunosorbent assay (ELISA), immunohistochemistry (IHC), affinity chromatography, etc.

EXAMPLES

Example 1: Using Somatic Mutations to Determine Tumor Site

Methods

Consider a sample from a patient with some type of cancer. The mutation screening of this sample identifies somatic mutations in $n$ genes $g_1, g_2, ..., g_n$. Assuming that somatic mutations occur independently, the probability that this patient has cancer of type $c$ is given by the following equation:

$$P(c|g_1, g_2, ..., g_n) = P_0(c)\ \Pi_i\ M(g_i|c)\ /\ \Sigma_t\ P_0(t)\ \Pi_i\ M(g_i|t) \qquad (1)$$

where the product is taken over all genes mutated in the sample ($i=1, 2, ..., n$) and the sum is taken over all cancer types $t$. $M(g|c)$ is the frequency of somatic mutations in gene g in cancer type $c$. *See* FIG.3 (with mutation frequencies based on data from COSMIC [Catalogue of Somatic Mutations in Cancer] database). $P_o(c)$ is the *a priori* probability of cancer type c given that the patient has a cancer. *See* FIG.4 (with these *a priori* probabilities based on cancer incidences published by the American Cancer Society). It should be noted that for some cancers (such as ovarian and prostate cancers) incidences

are drastically different in males and females, therefore, Equation (1) may in some instances be used separately for males and females.

Using Equation (1), the probabilities were calculated for each cancer type $c$, and the cancer with the highest probability was designated the most likely cancer type in the patient. Such a patient may be examined by available diagnostic techniques for this cancer type. If the most likely cancer type is not confirmed, the cancer type with the second highest probability should be examined and so on.

Since some organs can develop cancers of different types (such as adenocarcinoma and squamous cell carcinoma in lung), one may calculate the probability $P(o)$ that the cancer has developed in organ $o$:

$$P(o|g_1, g_2, ..., g_n) = \Sigma_c\ P(c|g_1, g_2, ..., g_n) \qquad (2)$$

where the sum is over all cancer types $c$ of the organ $o$. Using Equation (2), the probabilities are calculated for each organ $o$, and the organ with the highest probability is the most likely cancer site in the patient. The patient may optionally be examined by additional diagnostic techniques to confirm this cancer site. If the most likely cancer site is not confirmed, the organ with the second highest probability may then be examined and so on.

Results

In order to evaluate the power of using mutations to determine the tumor site, we used three published studies (PMID: 17932254 , PMID: 18772397, PMID: 18772396) in which over 20,000 genes were sequenced in samples representing four cancers: 11 breast ductal carcinoma samples, 11 colon adenocarcinoma samples, 22 glioblastoma samples, and 24 pancreatic ductal carcinoma samples. We used these datasets as a validation dataset for our approach. In order to calculate the probabilities given by Equation (1) for these samples, we used two sets of genes.

The first set of genes represents all the genes with mutation frequency above 5% in one of 29 common cancer types. Using COSMIC database we identified 33 such genes:

**Table 2**

| | | |
|---|---|---|
| APC | KRAS | PRKDC |
| ATM | HRAS | PTEN |
| BRAF | NRA S | RB1 |
| BRCA1 | MAP2K4 | RET |
| BRCA2 | MET | SMAD4 |
| CDKN2A | MLH1 | SMO |
| CTNNB1 | MSH2 | STK11 |

(continued)

| EGFR | MSH6 | TAFI L |
|------|------|--------|
| FBXW7 | NF1 | TP53 |
| FGFR3 | NF2 | TRRAP |
| KIT | PIK3 CA | VHL |

Using this set of genes the following results were obtained:

| Cancer Type | Percent Correct 1 | Percent Correct 2 | Percent Wrong |
|-------------|-------------------|-------------------|---------------|
| Breast | 91 | 9 | 0 |
| Colon | 55 | 45 | 0 |
| Glioblastoma | 0 | 0 | 100 |
| Pancreatic | 42 | 29 | 29 |

"Percent Correct 1" is the percent of samples for which the cancer type with highest predicted probability coincided with the true cancer type of the sample, "Percent Correct 2" is the percent of samples for which cancer type with the second highest predicted probability coincided with the true cancer type of the sample, and "Percent Wrong" is the percent of samples for which cancers types with neither highest nor second highest predicted probabilities coincided with the true cancer type of the sample.

The second set of genes was based on the validation dataset. The set was composed of genes which satisfied the following conditions:

1. The gene should have two or more somatic mutations observed in samples form at least one cancer type.
2. Frequency of somatic mutations in the gene should be more than 5% in prevalence samples.
3. The gene should be known to be cancer-related.

17 genes satisfied these conditions:

**Table 3**

| AIM1 | FLJ13479 | RB1 |
|------|----------|-----|
| APC | IDH1 | SMAD4 |
| CDKN2A | KRAS | TGFBR2 |
| EGFR | PIK3CA | TNN |
| FBN2 | PIK3R1 | TP53 |
| FBXW7 | PTEN | |

Using this list of genes thus gave better prediction accuracy, as shown in the following table:

| Cancer Type | % Correct 1 | % Correct 2 | % Wrong |
|-------------|-------------|-------------|---------|
| Breast | 91 | 9 | 0 |
| Colon | 100 | 0 | 0 |
| Glioblastoma | 41 | 50 | 9 |
| Pancreatic | 88 | 12 | 0 |

**Method variations**

Some modifications to the above approach may be applied individually or in combination to improve results or under certain circumstances.

1. In Equation (1), rather than using somatic mutation frequencies of individual genes, one can use frequencies of somatic mutations in certain combinations of genes. For examples, rather than using individual mutation frequencies for TP53 and KRAS genes, one can use frequencies of events when both genes are mutated or when either of them is mutated.

2. Equation (1) is relying on the presence of somatic mutations in a set of genes. One can also utilize the absence of mutations in addition to utilizing the presence of mutations. In this case instead of Equation (1) one would use the following equation:

$$P(c|g_1, g_2, ...,g_n) = P_0(c)\ \Pi_i\ M(g_i|c)\ \Pi_j\ (1\text{-}M(g_i|c))\ /\ \Sigma_t\ P_0(t)\ \Pi_i\ M(g_i|t)\ \Pi_j\ (1\text{-}M(g_i|c))$$

where the product over j is a product over all the non-mutated genes in the set.

3. Many cancer-related genes have so called 'mutation hot spots' which are small areas where the majority of somatic mutations occur. These areas can be easily identified from COSMIC database. Rather than utilizing any somatic mutations in a gene, one can restrict the approach to 'mutation hot spots' only.

4. A priori probabilities $P_0(c)$ in Equation (1) can incorporate patient's personal information known to affect cancer risk. For example, females with germline mutations in BRCA1 or BRCA2 genes are at high risk of developing breast and ovarian cancers.

Example 2: Using Somatic Mutations to Detect Presence of Cancer

**Method**

Since somatic mutations are very specific to cancer or pre-cancerous conditions, the main performance measure of using mutation screening of a set of genes is its sensitivity. The sensitivity of screening for any cancer depends on sensitivities within individual cancers as well as on the incidences of the cancers. The sensitivity was defined by the following equation:

$$S = \Sigma_t\ P_0(t)S(t) \qquad (4)$$

where $S(t)$ was the sensitivity within cancer type $t$. $S(t)$ was defined as the percentage of patients with somatic mutations in one or more genes within a predefined set of one or more genes.

The following algorithm was used to define a small set of genes with high sensitivity:

1. Started with all available samples and an empty list of genes.
2. Within current set of samples, found the gene with highest sensitivity calculated according to Equation (4). This gene was added to the list of genes.
3. Repeated Steps 1 & 2 until the combined sensitivity of the resultant list of genes was high enough. If more sensitivity is desired one may proceed to Step 4.
4. Reduce the set of samples by eliminating all samples which have mutations in any of the genes from the current list.
5. Return to Step 2 to further increase sensitivity.

**Results**

The same validation dataset described above was used. The list of genes in the order they were define by the above algorithm is shown below, with the cumulative sensitivity as a function of the number of the genes in the list presented in FIG.1:

**Table 4**

| TP53 |
|------|
| KRAS |
| APC |
| EGFR |
| PTEN |

## Method variations

Some modifications to the above approach may be applied individually or in combination to improve results or under certain circumstances.

1. Rather than relaying on any somatic mutations in a gene, one can restrict the approach to mutation hot spots only.
2. The approach can be used not only for detecting any cancer but for detecting certain groups of cancers including individual cancer types (e.g., screening individuals at high risk of certain cancers).
3. If one needs to distinguish between pre-cancerous benign tumors and malignant cancers, only genes with mutations in cancers but not in benign tumors can be used.

Example 3: Detecting Mutations in Exosomes

### Method

To confirm our ability to detect cancer-related mutations in serum exosomes, cell culture supernatants (1-10ml from ovarian and colon cancer cell lines) or ovarian and colon cancer patient serum samples (1-3ml) were used to prepare exosomes by high-speed centrifugation. Total RNA was extracted from exosomal pellets and converted to cDNA by standard methods. PCR amplicons for a set of mutation hot spots in *TP53, KRAS, EGFR* and *APC* were designed and optimized for multiplexing. Exosomal cDNA was preamplified with a multiplex of all amplicons. The pre-amplification product was split into separate reactions and re-amplified with the individual target amplicons. Re-amplification primers were synthesized with tails for dye-primer sequencing. Individual PCR products were sequenced by dye-primer chemistry to identify particular mutations.

### Results

Mutations were found in exosomes harvested from cell lines as follows:

**Table 5**

| Gene | Cell Line | DNA mutation | Cell Line RNA | Exosomal RNA (no preamp) | Exosomal RNA (preamp) |
|------|-----------|--------------|---------------|--------------------------|------------------------|
| TP53 | T47D | L194F | L194F | na | L194F |
| TP53 | OVCA5 | WT | Exon6/7 splice variant | na | Exon6/7 splice variant |
| TP53 | HT29 | R273H | nd | nd | R273H |
| KRAS | OVCA5 | G12V | G12V | na | G12V |
| KRAS | HCT15 | G13D | nd | nd | G13D |
| na = no available sequence, nd = not done | | | | | |

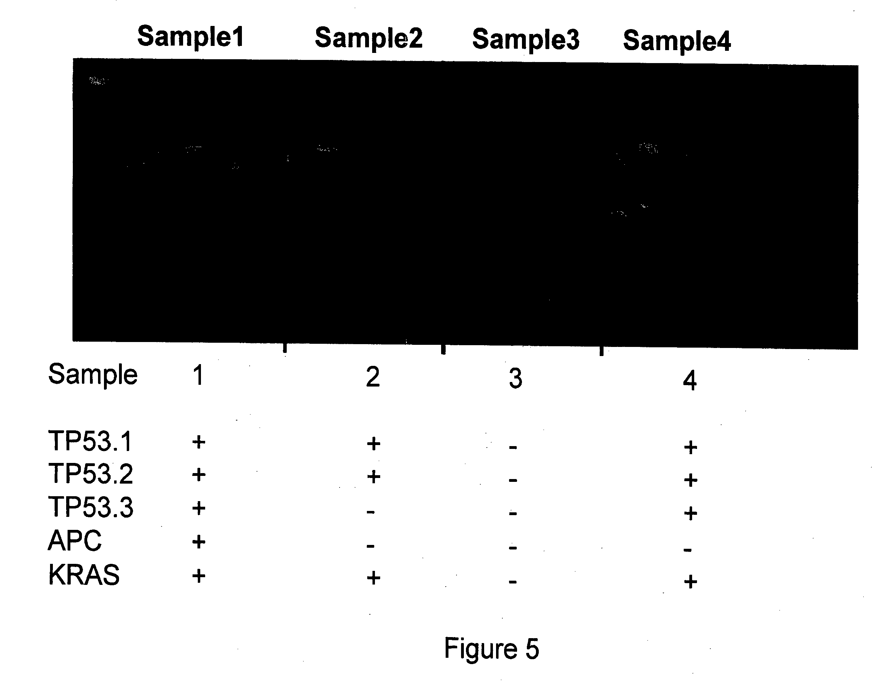

Mutations were found in cancer serum samples as follows (gels showing mutations in ovarian cancer serum shown in FIG.5):

**Table 6**

| Cancer | Ovarian | Colon | All |
|---|---|---|---|
| Samples tested | 9 | 54 | 65 |
| Positive amplification | 24 (53%) | 81 (30%) | 105 (33%) |
| Sequence positive | 100% | 89% | 91% |
| # mutations | 1 | 10 | 11 |
| # mutant samples | 1 (11%) | 8 (15%) | 9 (14%) |

Examples of important mutations in genes listed in Table 1 are shown below in Table 7:

**Table 7**

| Gene | Hot Spot | Amino Acid Change | cDNA pos. |
|---|---|---|---|
| TP53 | 1 | R175H/L;C176F/Y | c.524;C527 |
| TP53 | 2 | R248W/G | c742;c743 |
| TP53 | 3 | R273C;R273H/L | c817;c818 |
| APC | | R1450* | c.4348 |
| KRAS | | G12C/S/R;G12D/V/A | c.34;c35 |
| BRAF | | V800E | c.1799 |
| EGFR | | L858R | not published |

Examples of important mutations found in cancer serum exosomes in Table 1 genes are shown below in Table 8:

**Table 8**

| Gene (hotspot) | Mutations | aa | aaChange | Codon Change | Tissues | Myr Sample ID |
|---|---|---|---|---|---|---|
| TP53 hs2 and 3 | G>A(homo) | | | CTG->CTA | | |
| TP53 hs2 and 3 | L265L | 265 | L265L | | Liver, Stomach | 1 |
| TP53 hs2 and 3 | A>G(homo) | | | AAC->AGC | | |
| TP53 hs2 and 3 | N239S | 239 | N239S | | Colon | 1 |
| TP53 hs2 and 3 | G>T(homo) | | | AGG->AGT | | |
| TP53 hs2 and 3 | R249S | 249 | R249S | | Colorectum | 1,2 |
| TP53 hs2 and 3 | | | | | | 2 |
| TP53 hs2 and 3 | G>A (Homo) | | | CGT->CAT | | |
| TP53 hs2 and 3 | | | | | | |
| TP53 hs2 and 3 | | | | | | |
| TP53 hs2 and 3 | R273H | 273 | R273H | | Colon | 1 |
| TP53 hs2 and 3 | | | | | | |
| TP53 hs2 and 3 | | | | | | |
| TP53 hs2 and 3 | G>A (homo) | | | GGC->AGC | | 3 |
| TP53 hs2 and 3 | | | | | | |
| TP53 hs2 and 3 | G245S | 245 | G245S | | Colon | |

(continued)

| Gene (hotspot) | Mutations | aa | aaChange | Codon Change | Tissues | Myr Sample ID |
|---|---|---|---|---|---|---|
| TP53 hs2 and 3 | A>G(homo) | | | ACA->GCA | Bladder, Breast, Hematopoietic, Lung and Skin | 4 |
| TP53 hs2 and 3 | T256A | 256 | T256A | | | |
| TP53 hs2 and 3 | A>G(homo) | | | AGA->AGG | Unspecified urinary organ; Renal pelvis | 5 |
| TP53 hs2 and 3 | R280R | 280 | R280R | | | |
| TP53 hs2 and 3 | T>C(homo) | | | CCT->CCC | Breast, Esophagus, Skin | 5 |
| TP53 hs2 and 3 | P278P | 278 | P278P | | | |
| TP53 hs1 | C>A het | | | CCC->CAC | Colon | 2 |
| TP53 hs1 | P151H | 151 | P151H | | | |
| TP53 hs1 | C>T(homo) | | | CTT->TTT | | 6 |
| TP53 hs1 | L194F | 194 | L194F | | Colon | 7 |
| APC | A>G (homo) | | | CGA->CGG | | 8 |
| APC | R1450R | 1450 | R1450R | | | |
| APC | C>T Het | | | GAT->GAC | | 9 |
| APC | D1425D | 1425 | D1425D | | | |
| KRAS | T>C (het) | | | CTT->CTC | | 10 |
| KRAS | L6L | 6 | L6L | | | |
| TP53hs2-3F3R3 | G>A | | | GGC->GAC | Colon | 11 |
| | | 245 | G245D | | | |
| KRASF4R4 | G>A(het) and (homo) | | | GGT->GAT | | 12 |
| | | 12 | G12D | | | |
| TP53hs2-3F3R3 | G>A(het) | | | CTG->CTA | | 13 |
| | | 265 | L265L | | | |

Although the foregoing invention has been described in some detail by way of illustration and example for purposes of clarity of understanding, it will be clear to those skilled in the art that certain changes and modifications may be practiced within the scope of the appended claims.

**Claims**

1. A method of determining the likelihood a patient has a particular cancer comprising:

(1) analyzing in a bodily fluid sample a panel of genes comprising the *APC, EGFR, KRAS, PTEN,* and *TP53* genes;
(2) determining whether each gene in said panel has a mutation;
(3) calculating a likelihood said patient has cancer $c_1$ using the formula: $P(c_1|g_1, g_2, ..., g_n) = P_0(c_1) \Pi_i M(g_i|c_1) / \Sigma_t P_0(t) \Pi_i M(g_i|t)$; wherein the product is taken over all genes in said panel mutated in the sample ($i=1, 2, ..., n$), the sum is taken over all cancer types $t$, $M(g|c_1)$ is the frequency of somatic mutations in gene $g$ in cancer type $c_1$, and $P_0(c_1)$ is the *a priori* probability of cancer $c_1$ given that the patient has a cancer.

**2.** The method of Claim 1, further comprising calculating a likelihood said patient has a second cancer $c_2$ using the formula: $P(c_2|g_1, g_2, ..., g_n) = P_0(c_2) \Pi_i M(g_i| c_2) / \Sigma_t P_0(t) \Pi_i M(g_i|t)$; wherein the product is taken over all genes in said panel mutated in the sample ($i=1,2,...,n$), the sum is taken over all cancer types $t$, $M(g| c_2)$ is the frequency of somatic mutations in gene g in cancer type $c_2$, and $P_0(c_2)$ is the *a priori* probability of cancer type $c_2$ given that the patient has a cancer.

**3.** The method of Claim 1 or 2, further comprising recommending, prescribing, ordering, or performing a test for the presence of cancer $c_1$ in said patient.

**4.** The method of Claim 3, wherein said test for the presence of cancer $c_1$ is recommended, prescribed, ordered, or performed if the calculated likelihood said patient has said cancer $c_1$ is above a threshold value.

**5.** The method of Claim 3, wherein said test for the presence of cancer $c_1$ is recommended, prescribed, ordered, or performed if the calculated likelihood said patient has said cancer $c_1$ is higher than the calculated likelihood said patient has cancer $c_2$.

**6.** The method of Claim 3, further comprising recommending, prescribing, ordering, or performing a test for the presence of cancer $c_2$ in said patient if said test for the presence of cancer $c_1$ does not indicate the presence of cancer $c_1$.

**7.** A method of detecting mutations comprising:

(1) analyzing in a bodily fluid sample from a human subject a panel of genes consisting of between 5 and 5,000 genes, wherein said panel comprises at least five genes chosen from the group consisting of the genes listed in Table 1 and at least the *APC, EGFR, KRAS, PTEN,* and *TP53* genes; and
(2) determining whether any of the genes of said panel harbors a mutation.

**8.** The method of Claim 7, wherein said panel comprises the genes listed in Table 3, the genes listed in Table 2 or the genes listed in Table 1.

**9.** The method of Claim 7, wherein said *APC, EGFR, KRAS, PTEN,* and *TP53* genes constitute at least 10% of said panel.

**10.** A method of detecting cancer comprising:

(1) analyzing a panel of genes comprising the *APC, EGFR, KRAS, PTEN,* and *TP53* genes in a bodily fluid sample; and
(2) determining whether any of the *APC, EGFR, KRAS, PTEN,* or *TP53* genes harbors a mutation;

wherein said mutation indicates the presence of cancer.

**11.** The method of Claim 10, wherein said mutation is selected from the group consisting of those listed in Table 7 and/or Table 8.

**12.** The method of Claim 10 or 11, wherein said panel comprises the genes listed in Table 3, the genes listed in Table 2 or the genes listed in Table 1.

**13.** The method of any one of Claims 10 to 12, wherein said bodily fluid sample is a blood sample.

**14.** The method of Claim 13, wherein said blood sample is a plasma sample or a serum sample.

**15.** The method of any one of Claims 1 to 14, wherein detecting a mutation or determining whether a gene harbors a mutation comprises

(i) analyzing an mRNA molecule from said sample or analyzing a DNA molecule synthesized using said mRNA molecule as a template;
(ii) analyzing a nucleic acid from said sample by a technique chosen from resequencing, TaqMan™, microarray analysis, and FISH; or
(iii) analyzing a nucleic acid deriving from an extracellular vesicle.

**16.** A kit comprising reagents for analyzing a panel of genes consisting of between 5 and 5,000 genes, wherein said kit comprises oligonucleotide probes and/or primers which detect mutations in at least the *APC, EGFR, KRAS, PTEN,* and *TP53* genes.

**17.** The kit of Claim 16, wherein said genes are the genes listed in Table 3, the genes listed in Table 2 or the genes listed in Table 1.

**18.** The kit of Claim 16, wherein said *APC, EGFR, KRAS, PTEN,* and *TP53* genes constitute at least 10% of the genes that may be analyzed in said kit.

**Patentansprüche**

**1.** Verfahren zur Bestimmung der Wahrscheinlichkeit, dass ein Individuum einen bestimmten Krebs hat, wobei das Verfahren umfasst:

(1) Analysieren einer Gruppe von Genen in einer Körperflüssigkeitsprobe, wobei die Gruppe von Genen die Gene *APC, EGFR, KRAS, PTEN* und *TP53* umfasst;

(2) Bestimmen, ob jedes der Gene in der Gruppe eine Mutation hat;

(3) Berechnen der Wahrscheinlichkeit, dass der Patient einen Krebs $c_1$ hat unter Verwendung der Formel: $P(c_1|g_1, g_2, ..., g_n) = P_0(c_1) \prod_i M(g_i| c_1) / \sum_t P_0(t) M(g_i|t)$, wobei das Produkt über alle Gene in der Gruppe, die in der Probe mutiert sind ($i=1, 2, ..., n$), genommen wird, die Summe über alle Krebsarten $t$ genommen wird, $M(g| c_1)$ die Häufigkeit der somatischen Mutationen in Gen $g$ in einer Krebsart $c_1$ ist, und $P_0(c_1)$ die a *priori*-Wahrscheinlichkeit des Krebses $c_1$ ist, vorausgesetzt, dass das Individuum einen Krebs hat.

**2.** Verfahren nach Anspruch 1, das zudem das Berechnen der Wahrscheinlichkeit umfasst, dass das Individuum einen zweiten Krebs $c_2$ hat, unter Verwendung der Formel $P(c_2|g_1, g_2, ..., g_n) = P_0(c_2) \prod_i M(g_i| c_2) / \sum_t P_0(t) \prod_i M(g_i|t)$; wobei das Produkt über alle Gene in der Gruppe, die in der Probe mutiert sind ($i=1, 2, ..., n$), genommen wird, die Summe über alle Krebsarten $t$ genommen wird, $M(g| c_2)$ die Häufigkeit der somatischen Mutationen in Gen $g$ in einer Krebsart $c_2$ ist, und $P_0(c_2)$ die a *priori*-Wahrscheinlichkeit der Krebsart $c_2$ ist, vorausgesetzt, dass das Individuum einen Krebs hat.

**3.** Verfahren nach Anspruch 1 oder 2, das zudem das Empfehlen, Verschreiben, Anordnen oder Durchführen eines Tests auf das Vorliegen des Krebses $c_1$ bei dem Individuum umfasst.

**4.** Verfahren nach Anspruch 3, wobei der Test auf das Vorliegen des Krebses $c_1$ empfohlen, verschrieben, angeordnet oder durchgeführt wird, wenn die berechnete Wahrscheinlichkeit, dass das Individuum den Krebs $c_1$ hat, über einem Schwellenwert ist.

**5.** Verfahren nach Anspruch 3, wobei der Test auf das Vorliegen des Krebses $c_1$ empfohlen, verschrieben, angeordnet oder durchgeführt wird, wenn die berechnete Wahrscheinlichkeit, dass das Individuum den Krebs $c_1$ hat, höher ist als die berechnete Wahrscheinlichkeit, dass das Individuum den Krebs $c_2$ hat.

**6.** Verfahren nach Anspruch 3, das zudem das Empfehlen, Verschreiben, Anordnen oder Durchführen eines Tests auf das Vorliegen des Krebses $c_2$ bei dem Individuum umfasst, wenn der Test auf das Vorliegen des Krebses $c_1$ nicht das Vorliegen des Krebses $c_1$ anzeigt.

**7.** Verfahren zum Nachweis von Mutationen, das umfasst:

(1) Analysieren einer Gruppe von Genen in einer Körperflüssigkeitsprobe eines menschlichen Individuums, wobei die Gruppe von Genen aus zwischen 5 bis 5000 Genen besteht, wobei die Gruppe mindestens fünf Gene umfasst, die ausgewählt sind aus der Gruppe bestehend aus den in Tabelle 1 aufgeführten Genen, und mindestens den Genen *APC, EGFR, KRAS, PTEN* und *TP53;* und

(2) Bestimmen, ob eines der Gene der Gruppe eine Mutation aufweist.

**8.** Verfahren nach Anspruch 7, wobei die Gruppe die in Tabelle 3 aufgeführten Gene, die in Tabelle 2 aufgeführten Gene oder die in Tabelle 1 aufgeführten Gene umfasst.

**9.** Verfahren nach Anspruch 7, wobei die Gene *APC, EGFR, KRAS, PTEN* und *TP53* mindestens 10% der Gruppe ausmachen.

**10.** Verfahren zum Nachweis von Krebs, das umfasst:

(1) Analysieren einer Gruppe von Genen in einer Körperflüssigkeitsprobe, wobei die Gruppe von Genen die Gene *APC, EGFR, KRAS, PTEN* und *TP53* umfasst; und
(2) Bestimmen, ob eines der Gene *APC, EGFR, KRAS, PTEN* und *TP53* eine Mutation aufweist;

wobei die Mutation das Vorliegen von Krebs anzeigt.

**11.** Verfahren nach Anspruch 10, wobei die Mutation ausgewählt ist aus der Gruppe bestehend aus denen, die in Tabelle 7 und/oder Tabelle 8 aufgeführt sind.

**12.** Verfahren nach Anspruch 10 oder 11, wobei die Gruppe die in Tabelle 3 aufgeführten Gene, die in Tabelle 2 aufgeführten Gene oder die in Tabelle 1 aufgeführten Gene umfasst.

**13.** Verfahren nach einem der Ansprüche 10 bis 12, wobei die Körperflüssigkeitsprobe eine Blutprobe ist.

**14.** Verfahren nach Anspruch 13, wobei die Blutprobe eine Plasmaprobe oder eine Serumprobe ist.

**15.** Verfahren nach einem der Ansprüche 1 bis 14, wobei das Nachweisen einer Mutation oder das Bestimmen, ob ein Gen eine Mutation aufweist, umfasst:

(i) Analysieren eines mRNA-Moleküls der Probe oder Analyiseren eines DNA-Moleküls, das unter Verwendung des mRNA-Moleküls als Template synthetisiert wurde,
(ii) Analysieren einer Nucleinsäure der Probe mittels einer Technik, die ausgewählt ist aus Resequenzierung, TaqMan™, Microarray-Analyse und FISH; oder
(iii) Analysieren einer Nucleinsäure, die aus einem extrazellulären Vesikel stammt.

**16.** Kit, das Reagenzien für das Analysieren einer Gruppe von Genen umfasst, wobei die Gruppe von Genen aus zwischen 5 bis 5000 Genen besteht, wobei das Kit Oligonucleotidsonden und/oder -Primer umfasst, die Mutationen in mindestens den Genen *APC, EGFR, KRAS, PTEN* und *TP53* nachweisen.

**17.** Kit nach Anspruch 16, wobei die Gene die in Tabelle 3 aufgeführten Gene, die in Tabelle 2 aufgeführten Gene oder die in Tabelle 1 aufgeführten Gene sind.

**18.** Kit nach Anspruch 16, wobei die Gene *APC, EGFR, KRAS, PTEN* und *TP53* mindestens 10% der Gene, die mit dem Kit analyisiert werden können, ausmachen.

## Revendications

**1.** Méthode de détermination de la probabilité selon laquelle un patient a un cancer particulier comprenant :

(1) l'analyse dans un échantillon de fluide corporel d'un panel de gènes comprenant les gènes *APC, EGFR, KRAS, PTEN,* et *TP53* ;
(2) la détermination pour chaque gène dudit panel de la présence ou non d'une mutation ;
(3) le calcul d'une probabilité selon laquelle ledit patient a un cancer $c_1$ à l'aide de la formule :

$P(c_1|g_1, g_2, ..., g_n) = P_0(c_1) \Pi_i M(g_i| c_1) / \Sigma_t P_0(t) \Pi_i M(g_i|t)$ ; dans laquelle le produit est calculé sur tous les gènes dudit panel mutés dans l'échantillon (*i=1, 2, ..., n*), la somme est calculée sur tous les types de cancers *t*, $M(g| c_1)$ est la fréquence des mutations somatiques du gène *g* dans le type de cancer $c_1$, et $P_0(c_1)$ est la probabilité *a priori* de cancer $c_1$ étant donné que le patient a un cancer.

**2.** Méthode selon la revendication 1, comprenant en outre le calcul d'une probabilité selon laquelle ledit patient a un second cancer $c_2$ à l'aide de la formule : $P(c_2|g_1, g_2, ..., g_n) = P_0(c_2) \Pi_i M(g_i| c_2) / \Sigma_t P_0(t) \Pi_i M(g_i|t)$ ; dans laquelle le produit est calculé sur tous les gènes dudit panel mutés dans l'échantillon (*i=1, 2, ..., n*), la somme est calculée sur tous les types de cancers $t$, $M(g| c_2)$ est la fréquence des mutations somatiques du gène $g$ dans le type de cancer $c_2$, et $P_0(c_2)$ est la probabilité *a priori* de cancer de type $c_2$ étant donné que le patient a un cancer.

3. Méthode selon la revendication 1 ou 2, comprenant en outre la recommandation, la prescription, l'ordonnance, ou l'exécution d'un test de dépistage du cancer $c_1$ chez ledit patient.

4. Méthode selon la revendication 3, dans laquelle ledit test de dépistage du cancer $c_1$ est recommandé, prescrit, ordonné, ou exécuté si la probabilité calculée selon laquelle ledit patient a ledit cancer $c_1$ est supérieure à une valeur seuil.

5. Méthode selon la revendication 3, dans laquelle ledit test de dépistage du cancer $c_1$ est recommandé, prescrit, ordonné, ou exécuté si la probabilité calculée selon laquelle ledit patient a ledit cancer $c_1$ est supérieure à la probabilité calculée selon laquelle ledit patient a un cancer $c_2$.

6. Méthode selon la revendication 3, comprenant en outre la recommandation, la prescription, l'ordonnance, ou l'exécution d'un test de dépistage du cancer $c_2$ chez ledit patient si ledit test de dépistage du cancer $c_1$ n'indique pas la présence du cancer $c_1$.

7. Méthode de détection de mutations comprenant :

(1) l'analyse dans un échantillon de fluide corporel provenant d'un sujet humain d'un panel de gènes comprenant entre 5 et 5000 gènes, dans laquelle ledit panel comprend au moins cinq gènes choisis dans le groupe constitué par les gènes listés dans le Tableau 1 et au moins les gènes *APC, EGFR, KRAS, PTEN,* et *TP53 ;* et
(2) la détermination pour l'un quelconque des gènes dudit panel de la présence ou non d'une mutation.

8. Méthode selon la revendication 7, dans laquelle ledit panel comprend les gènes listés dans le Tableau 3, les gènes listés dans le Tableau 2 ou les gènes listés dans le Tableau 1.

9. Méthode selon la revendication 7, dans laquelle lesdits gènes *APC, EGFR, KRAS, PTEN,* et *TP53* constituent au moins 10 % dudit panel.

10. Méthode de dépistage du cancer comprenant :

(1) l'analyse d'un panel de gènes comprenant les gènes *APC, EGFR, KRAS, PTEN,* et *TP53* dans un échantillon de fluide corporel ; et
(2) la détermination pour l'un quelconque des gènes *APC, EGFR, KRAS, PTEN,* et *TP53* de la présence ou non d'une mutation ;

dans laquelle ladite mutation indique la présence du cancer.

11. Méthode selon la revendication 10, dans laquelle ladite mutation est choisie dans le groupe constitué par celles listées dans le Tableau 7 et/ou dans le Tableau 8.

12. Méthode selon la revendication 10 ou 11, dans laquelle ledit panel comprend les gènes listés dans le Tableau 3, les gènes listés dans le Tableau 2 ou les gènes listés dans le Tableau 1.

13. Méthode selon l'une quelconque des revendications 10 à 12, dans laquelle ledit échantillon de fluide corporel est un échantillon de sang.

14. Méthode selon la revendication 13, dans laquelle ledit échantillon de sang est un échantillon de plasma ou un échantillon de sérum.

15. Méthode selon l'une quelconque des revendications 1 à 14, dans laquelle la détection d'une mutation ou la détermination de la présence ou non d'une mutation dans un gène comprend

(i) l'analyse d'une molécule d'ARNm provenant dudit échantillon ou l'analyse d'une molécule d'ADN synthétisée à l'aide de ladite molécule d'ARNm en tant que matrice ;
(ii) l'analyse d'un acide nucléique provenant dudit échantillon par une technique choisie parmi le reséquençage, TaqMan™, l'analyse sur micropuce, et FISH ; ou

(iii) l'analyse d'un acide nucléique dérivant d'une vésicule extracellulaire.

16. Kit comprenant des réactifs pour analyser un panel de gènes comprenant entre 5 et 5000 gènes, dans lequel ledit kit comprend des sondes et/ou des amorces oligonucléotidiques qui détectent des mutations dans au moins les gènes *APC, EGFR, KRAS, PTEN,* et *TP53.*

17. Kit selon la revendication 16, dans lequel lesdits gènes sont les gènes listés dans le Tableau 3, les gènes listés dans le Tableau 2 ou les gènes listés dans le Tableau 1.

18. Kit selon la revendication 16, dans lequel lesdits gènes *APC, EGFR, KRAS, PTEN,* et *TP53* constituent au moins 10 % des gènes qui peuvent être analysés dans ledit kit.

Figure 1

```
┌─────────────────────────────────────────────┐
│  Cytokeratin 7 (−) / Cytokeratin 20 (−)      │────┐ ┌─────┐
└─────────────────────────────────────────────┘    └─│ 110 │
                                                      └─────┘
```

| PSA | Hep Par 1 | CD10 |
|-----|-----------|------|
| PSAP | AFP | Vimentin |
| PSMA | CAM 5.2 | RCC |
| | | EMA |

210

**Prostate Adenocarcinoma**

PSA (+) or

PSAP (+) or

PSMA (+)

310

**Hepatocellular Carcinoma**

Hep Par 1 (+) or

AFP (+) or

CAM 5.2 (+)

311

**Renal Cell Carcinoma, clear cell type**

CD10 (+) or

Vimentin (+) or

RCC (+) or

EMA

312

Figure 2A

Cytokeratin 7 (−) / Cytokeratin 20 (+) — 120

Ae1/3
CAM 5.2
CK19
CEA (polyclonal)
EMA — 220

320 — Colon
Adenocarcinoma

Ae1/3 (+) or

CAM 5.2 (+) or

CK19 (+) or

CEA (polyclonal) (+) or

EMA (+)

420

* Imaging /
Endoscopy

Figure 2B

EP 2 438 197 B1

Cytokeratin 7 (+) / Cytokeratin 20 (−)  130

230

Thyroglobulin
Calictonin

Chromogranin
Synaptophysin
CD56 (NCAM)
Leu7
CK5/6
CEA
Mucicarmine
B72.3
Leu M1 (CD15)

Vimentin

PSA
PSAP
PSMA

HER2
GCDFP-15

Calretinin
HBME-1
CK5/6
Mesothelin

CK5/6
CEA

Prostate  Thyroid  Breast  332  Mesothelioma  Endometrial Cancer  Cervical Cancer

330  331  430 *Imaging  333  334  335

Lung

Small Cell / Neuroendocrine Carcinoma
Chromogranin (+) or
Synaptophysin (+) or
CD56 (NCAM) (+) or
Leu7
336

Squamous Cell Carcinoma
CK5/6 (+)
337

AdenoCarcinoma
CEA (+) or
Mucicarmine (+) or
B72.3 (+) or
Leu M1 (CD15)
338

Figure 2C

Cytokeratin 7 (+) / Cytokeratin 20 (+)  — 140

| CA-125 Mesothelin | 34BE12 Villin CA-125 | Uroplakin III | | CD10 | — 240 |

**Ovarian Carcinoma**

CA-125 (+) or Mesothelin (+)

— 340

**Cholangio Carcinoma**

34βE12 (+) or Villin (+) or CA-125 (+)

— 341

**Urothelial Carcinoma**

Uroplakin III (+)

— 342

**Papillary-Type Renal Cell Carcinoma**

CD10 (+)

— 343

**Chromophobe Renal Cell Carcinoma**

No marker

— 344

Figure 2D

| COSMIC Mutation Frequencies | | | | | | | | | | | | | | |
|---|---|---|---|---|---|---|---|---|---|---|---|---|---|---|
| | Lung | | | | Ovary | | | | Prost. | Colon | Brain | Skin | Breast | Kidney | |
| Gene Abbrev. | ADC | SCC | SCLC | Lg | Ser. | Muc. | Clr Cell | Endo. | ADC | ADC | Glio. | Melan. | Duct. | Clr Cell | Papill. |
| APC | 1 | 4 | 1 | 15 | 1 | 1 | 1 | 8 | 7 | 29* | 1 | 2 | 12 | 0.5 | 1 |
| ATM | 13 | 0.5 | 0.5 | 6 | 0.5 | 0.5 | 0.5 | 0.5 | 0.5 | 7 | 9 | 0.5 | 0.4 | 2 | 0.5 |
| BRAF | 3 | 3 | 1 | 1 | 2 | 2 | 2 | 9 | 7 | 14 | 4 | 44* | 1 | 1 | 1 |
| BRCA1 | 0.5 | 0.5 | 0.5 | 0.5 | 4 | 0.5 | 0.5 | 2 | 0.5 | 0.5 | 0.5 | 0.5 | 15 | 0.5 | 0.5 |
| BRCA2 | 0.5 | 0.5 | 0.5 | 0.5 | 9 | 0.5 | 0.5 | 0.5 | 0.5 | 4 | 0.5 | 2 | 5 | 1 | 0.5 |
| CDKN2A | 22 | 23 | 2 | 25 | 6 | 20 | 21 | 16 | 4 | 2 | 23 | 29 | 11 | 7 | 6.5 |
| CTNNB1 | 5 | 1 | 1 | 0.5 | 0.3 | 2 | 0.5 | 25 | 7 | 7 | 0.5 | 6 | 0.3 | 1 | 0.5 |
| EGFR | 39* | 4 | 5 | 4 | 0.2 | 0.5 | 0.5 | 0.5 | 6 | 0.2 | 4 | 1 | 1 | 2 | 0.5 |
| FBXW7 | 0.5 | 0.5 | 1 | 0.5 | 4 | 0.5 | 0.5 | 3 | 1 | 6 | 0.5 | 2 | 0.5 | 0.1 | 0.5 |
| FGFR3 | 1 | 0.5 | 0.5 | 6 | 0.5 | 0.5 | 0.5 | 0.5 | 0.5 | 0.5 | 0.5 | 0.5 | 0.5 | 0.5 | 0.5 |
| HRAS | 0.2 | 1 | 0.3 | 4 | 0.5 | 0.5 | 0.5 | 0.5 | 6 | 0.5 | 0.2 | 1 | 0.5 | 0.3 | 0.5 |
| KIT | 0.5 | 0.5 | 2 | 0.5 | 0.5 | 0.5 | 0.5 | 0.5 | 0.5 | 0.5 | 0.3 | 9 | 0.5 | 0.5 | 68* |
| KRAS | 22 | 5 | 1 | 21 | 8 | 43 | 8 | 7 | 8 | 37 | 1 | 2 | 3 | 0.2 | 2 |
| MAP2K4 | 3 | 3 | 3 | 4 | 5 | 2 | 2 | 2 | 2 | 12 | 1 | 2 | 4 | 2 | 2 |
| MET | 2 | 2 | 8 | 0.5 | 0.5 | 0.5 | 0.5 | 0.5 | 0.5 | 0.5 | 2 | 0.5 | 1 | 5 | 4 |
| MLH1 | 0.5 | 0.5 | 0.5 | 0.5 | 0.5 | 0.5 | 0.5 | 0.5 | 0.5 | 7 | 0.5 | 3 | 0.5 | 0.5 | 0.5 |
| MSH2 | 0.5 | 0.5 | 0.5 | 0.5 | 0.5 | 0.5 | 0.5 | 0.5 | 0.5 | 12 | 0.5 | 2 | 3 | 0.5 | 0.5 |
| MSH6 | 0.5 | 0.5 | 0.5 | 8 | 0.5 | 0.5 | 0.5 | 0.5 | 0.5 | 0 | 15 | 3 | 0.5 | 0.5 | 0.5 |
| NF1 | 0.5 | 0.5 | 0.5 | 0.5 | 0.5 | 0.5 | 0.5 | 0.5 | 0.5 | 15 | 0.4 | 2 | 0.5 | 1 | 0.5 |
| NF2 | 0.5 | 0.5 | 0.5 | 0.5 | 0.5 | 0.5 | 0.5 | 0.5 | 0.5 | 0.5 | 0.2 | 6 | 5 | 0.5 | 0.5 |
| NRAS | 1 | 0.3 | 0.2 | 7 | 0.5 | 0.5 | 0.5 | 0.5 | 2 | 2 | 1 | 21 | 0.5 | 0.2 | 0.5 |
| PIK3CA | 3 | 3 | 8 | 8 | 2 | 6 | 25 | 10 | 2 | 21 | 5 | 3 | 24 | 3 | 3 |
| PRKDC | 11 | 0.5 | 0.5 | 0.5 | 0.5 | 0.5 | 0.5 | 0.5 | 0.5 | 0.5 | 0.5 | 57* | 0.5 | 0.5 | 0.5 |
| PTEN | 1 | 8 | 13 | 12 | 3 | 16 | 3 | 19 | 13 | 14 | 20 | 18 | 4 | 4 | 2 |
| RB1 | 4 | 6 | 51 | 1 | 1 | 1 | 1 | 1 | 11 | 2 | 10 | 10 | 12 | 4 | 1 |
| RET | 2 | 0.5 | 2 | 0.5 | 0.5 | 0.5 | 0.5 | 0.5 | 0.5 | 0.5 | 0.5 | 0.5 | 0.5 | 0.4 | 0.5 |
| SMAD4 | 7 | 5 | 1 | 0.5 | 4 | 0.5 | 5 | 0.5 | 0.5 | 7 | 0.5 | 0.5 | 3 | 0.5 | 0.5 |
| SMO | 0.5 | 0.5 | 0.5 | 0.5 | 0.5 | 0.5 | 0.5 | 0.5 | 0.5 | 21 | 0.5 | 0.5 | 0.5 | 0.5 | 0.5 |
| STK11 | 14 | 4 | 1 | 14 | 0.5 | 0.5 | 0.5 | 0.5 | 0.5 | 15 | 0.5 | 10 | 0.5 | 0.5 | 0.5 |
| TAF1L | 12 | 0.5 | 0.5 | 0.5 | 5 | 0.5 | 0.5 | 0.5 | 0.5 | 0.5 | 22 | 0.5 | 0.5 | 0.5 | 0.5 |
| TP53 | 59 | 55 | 74 | 38 | 5 | 30 | 30 | 30 | 30 | 70 | 55 | 27 | 50 | 3 | 30 |
| TRRAP | 0.5 | 0.5 | 0.5 | 0.5 | 10 | 0.5 | 0.5 | 0.5 | 0.5 | 0.5 | 0.5 | 0.5 | 0.5 | 0.5 | 0.5 |
| VHL | 0.5 | 0.5 | 0.5 | 0.5 | 0.5 | 0.5 | 0.5 | 0.5 | 0.5 | 0.5 | 0.5 | 0.5 | 0.5 | 46* | 1 |

\* Indicates cancer where this gene is particularly predictive (i.e., mutated particularly often)

**Figure 3-A**

| COSMIC Mutation Frequencies | | | | | | | | | | | | | |
|---|---|---|---|---|---|---|---|---|---|---|---|---|---|
| | Liver | Pancr. | Thyroid | | Bladd. | Oral | Stom. | Esoph. | Testis | Cervix | | Larynx | Gallblad. | Endomet. |
| Gene Abbrev. | HCC | Duct. | Medull. | Papill. | TCC | SCC | ADC | | | SCC | ADC | SCC | ADC | ADC |
| APC | 5 | 13 | 1 | 15 | 1 | 0.9 | 11 | 1 | 1 | 1 | 1 | 1 | 1. | 1 |
| ATM | 0.5 | 0.5 | 0.5 | 0.5 | 0.5 | 0.5 | 5 | 0.5 | 0.5 | 0.5 | 0.5 | 0.5 | 0.5 | 0.5 |
| BRAF | 2 | 2 | 25* | 47* | 1 | 0.3 | 1 | 2 | 1 | 0.3 | 3 | 1. | 11 | 1 |
| BRCA1 | 0.5 | 0.5 | 0.5 | 0.5 | 0.5 | 0.5 | 0.5 | 0.5 | 0.5 | 0.5 | 0.5 | 0.5 | 0.5 | 0.5 |
| BRCA2 | 1 | 2 | 0.5 | 0.5 | 0.5 | 0.5 | 0.5 | 6 | 0.5 | 0.5 | 0.5 | 0.5 | 0.5 | 0.5 |
| CDKN2A | 14 | 37 | 6.5 | 9 | 17 | 18 | 8 | 21 | 1.5 | 12 | 2.5 | 17 | 31 | 3 |
| CTNNB1 | 17 | 4 | 0.5 | 14 | 2 | 0.4 | 6 | 1 | 0.5 | 5 | 8 | 0.5 | 10 | 11 |
| EGFR | 1 | 1 | 0.5 | 3 | 0.5 | 3 | 1 | 1 | 0.5 | 0.5 | 0.5 | 4 | 0.5 | 0.5 |
| FBXW7 | 0.5 | 2 | 0.5 | 0.5 | 0.5 | 0.5 | 6 | 0.5 | 0.5 | 0.5 | 0.5 | 0.5 | 0.5 | 22 |
| FGFR3 | 0.5 | 0.5 | 0.5 | 0.5 | 50* | 32 | 0.5 | 0.5 | 0.5 | 2 | 0.5 | 0.5 | 0.5 | 0.5 |
| HRAS | 0.3 | 0.5 | 1 | 1 | 7 | 13 | 4 | 1 | 0.5 | 7 | 18 | 2 | 0.5 | 3 |
| KIT | 0.5 | 0.5 | 0.5 | 0.5 | 0.5 | 0.5 | 2 | 0.5 | 9 | 0.5 | 0.5 | 0.5 | 0.5 | 0.5 |
| KRAS | 3 | 71 | 0.7 | 2 | 3 | 2 | 6 | 4 | 5 | 2 | 13 | 2 | 28 | 15 |
| MAP2K4 | 2 | 5 | 2 | 2 | 12 | 2 | 0.4 | 2 | 5 | 2 | 2 | 2 | 2 | 2 |
| MET | 12 | 0.5 | 0.5 | 1 | 0.5 | 0.5 | 2 | 0.5 | 7 | 0.5 | 0.5 | 0.5 | 0.5 | 0.5 |
| MLH1 | 0.5 | 24 | 0.5 | 0.5 | 0.5 | 0.5 | 5 | 0.5 | 0.5 | 0.5 | 0.5 | 0.5 | 0.5 | 0.5 |
| MSH2 | 0.5 | 0.5 | 0.5 | 0.5 | 0.5 | 0.5 | 2 | 0.5 | 0.5 | 0.5 | 0.5 | 0.5 | 0.5 | 3 |
| MSH6 | 0.5 | 0.5 | 0.5 | 0.5 | 14 | 0.5 | 8 | 0.5 | 0.5 | 0.5 | 0.5 | 0.5 | 0.5 | 8 |
| NF1 | 0.5 | 0.5 | 0.5 | 0.5 | 0.5 | 0.5 | 5 | 0.5 | 0.5 | 0.5 | 0.5 | 0.5 | 0.5 | 0.5 |
| NF2 | 0.5 | 0.5 | 0.5 | 0.5 | 0.5 | 0.5 | 0.5 | 0.5 | 0.5 | 0.5 | 0.5 | 0.5 | 0.5 | 0.5 |
| NRAS | 5 | 1 | 0.5 | 4 | 3 | 0.3 | 2 | 0.3 | 3 | 3 | 0.5 | 1 | 1 | 0.5 |
| PIK3CA | 6 | 8 | 3 | 3 | 23 | 12 | 8 | 5 | 3 | 23 | 3 | 1.9 | 4 | 3 |
| PRKDC | 0.5 | 0.5 | 0.5 | 0.5 | 0.5 | 0.5 | 0.5 | 0.5 | 0.5 | 0.5 | 0.5 | 0.5 | 0.5 | 0.5 |
| PTEN | 5 | 2 | 2 | 2 | 7 | 1 | 7 | 1 | 2 | 2 | 7 | 23 | 2 | 34 |
| RB1 | 10 | 1 | 1 | 1 | 26 | 1 | 10 | 5 | 1 | 1 | 1 | 1 | 1 | 1 |
| RET | 0.5 | 0.5 | 40* | 0.5 | 0.5 | 0.5 | 0.5 | 0.5 | 0.5 | 0.5 | 0.5 | 0.5 | 0.5 | 0.5 |
| SMAD4 | 2 | 28 | 0.5 | 18 | 0.5 | 20 | 4 | 3 | 8 | 0.5 | 0.5 | 0.5 | 0.5 | 0.5 |
| SMO | 4 | 0.5 | 0.5 | 0.5 | 0.5 | 0.5 | 0.5 | 0.5 | 0.5 | 0.5 | 0.5 | 0.5 | 0.5 | 0.5 |
| STK11 | 0.5 | 4 | 0.5 | 0.5 | 0.5 | 0.5 | 2 | 4 | 2 | 5 | 0.5 | 0.5 | 0.5 | 0.5 |
| TAF1L | 0.5 | 0.5 | 0.5 | 0.5 | 0.5 | 0.5 | 0.5 | 0.5 | 0.5 | 0.5 | 0.5 | 0.5 | 0.5 | 0.5 |
| TP53 | 70 | 60 | 30 | 30 | 80 | 96.6 | 33 | 96 | 6 | 30 | 30 | 30 | 30 | 80 |
| TRRAP | 0.5 | 0.5 | 0.5 | 0.5 | 0.5 | 0.5 | 10 | 0.5 | 0.5 | 0.5 | 0.5 | 0.5 | 0.5 | 0.5 |
| VHL | 0.5 | 3 | 0.5 | 0.5 | 0.5 | 0.5 | 0.5 | 0.5 | 0.5 | 0.5 | 0.5 | 0.5 | 0.5 | 0.5 |
| * Indicates cancer where this gene is particularly predictive (i.e., mutated particularly often) | | | | | | | | | | | | | |

**Figure 3-B**

| | All | Male | Female | All (%) | Male (%) | Female (%) |
|---|---|---|---|---|---|---|
| **Lung** | 215020 | 114690 | 100330 | 14.96 | 15.39 | 14.50 |
| **Breast** | 184450 | 1990 | 182460 | 12.83 | 0.27 | 26.37 |
| **Prostate** | 186320 | 186320 | 0 | 12.96 | 25.00 | 0.00 |
| **Colorectal** | 148810 | 77250 | 71560 | 10.35 | 10.37 | 10.34 |
| **Pancreas** | 37680 | 18770 | 18910 | 2.62 | 2.52 | 2.73 |
| **Bladder** | 68810 | 51230 | 17580 | 4.79 | 6.87 | 2.54 |
| **Kidney** | 54390 | 33130 | 21260 | 3.78 | 4.45 | 3.07 |
| **Thyroid** | 37340 | 8930 | 28410 | 2.60 | 1.20 | 4.11 |
| **Brain** | 21810 | 11780 | 10030 | 1.52 | 1.58 | 1.45 |
| **Ovary** | 21650 | 0 | 21650 | 1.51 | 0.00 | 3.13 |
| **Melanoma** | 62480 | 34950 | 27530 | 4.35 | 4.69 | 3.98 |
| **Oral** | 35310 | 25310 | 10000 | 2.46 | 3.40 | 1.45 |
| **Liver** | 21370 | 15190 | 6180 | 1.49 | 2.04 | 0.89 |
| **Stomach** | 21500 | 13190 | 8310 | 1.50 | 1.77 | 1.20 |
| **Esophagus** | 16470 | 12970 | 3500 | 1.15 | 1.74 | 0.51 |
| **Lymphoma** | 74340 | 39850 | 34490 | 5.17 | 5.35 | 4.98 |
| **Myeloma** | 19920 | 11190 | 8730 | 1.39 | 1.50 | 1.26 |
| **Leukemia** | 44270 | 25180 | 19090 | 3.08 | 3.38 | 2.76 |
| **Testis** | 8090 | 8090 | 0 | 0.56 | 1.09 | 0.00 |
| **Endometrium** | 40100 | 0 | 40100 | 2.79 | 0.00 | 5.79 |
| **Cervix** | 11070 | 0 | 11070 | 0.77 | 0.00 | 1.60 |
| **Larynx** | 12250 | 9680 | 2570 | 0.85 | 1.30 | 0.37 |
| **Gallbladder** | 9520 | 4500 | 5020 | 0.66 | 0.60 | 0.73 |
| **Totals** | 1437180 | 745180 | 692000 | 94.14 | 94.50 | 93.75 |

# Figure 4

|            | Sample 1 | Sample 2 | Sample 3 | Sample 4 |
|------------|:--------:|:--------:|:--------:|:--------:|

| Sample   | 1 | 2 | 3 | 4 |
|----------|:-:|:-:|:-:|:-:|
| TP53.1   | + | + | − | + |
| TP53.2   | + | + | − | + |
| TP53.3   | + | − | − | + |
| APC      | + | − | − | − |
| KRAS     | + | + | − | + |

Figure 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61184685 A
- US 7442507 B
- US 7198923 B
- US 6420108 B
- US 5593839 A
- US 5795716 A
- US 5733729 A
- US 5974164 A
- US 6066454 A
- US 6090555 A
- US 6185561 A
- US 6188783 A
- US 6223127 A
- US 6229911 A
- US 6308170 A
- US 10197621 B
- US 20030097222 A
- US 10063559 B
- US 20020183936 A
- US 10065856 B
- US 20030100995 A
- US 10065868 B
- US 20030120432 A
- US 10423403 B
- US 20040049354 A

### Non-patent literature cited in the description

- **SETUBAL et al.** INTRODUCTION TO COMPUTATIONAL BIOLOGY METHODS. PWS Publishing Company, 1997
- COMPUTATIONAL METHODS IN MOLECULAR BIOLOGY. Elsevier, 1998
- **RASHIDI ; BUEHLER.** BIOINFORMATICS BASICS: APPLICATION IN BIOLOGICAL SCIENCE AND MEDICINE. CRC Press, 2000
- **OUELETTE ; BZEVANIS.** BIOINFORMATICS: A PRACTICAL GUIDE FOR ANALYSIS OF GENE AND PROTEINS. Wiley & Sons, Inc, 2001